(19) Europäisches Patentamt
European Patent Office
Office européen des brevets

(11) Veröffentlichungsnummer: **0 172 139**
**B1**

(12) # EUROPÄISCHE PATENTSCHRIFT

(45) Veröffentlichungstag der Patentschrift: **14.09.88**

(21) Anmeldenummer: **85810353.4**

(22) Anmeldetag: **31.07.85**

(51) Int. Cl.⁴: **D 21 D 3/00,** D 21 H 3/02, C 07 C 69/24, C 07 C 69/54, C 07 C 69/86, C 07 C 103/375, C 07 C 103/60, C 07 C 125/067, C 07 C 127/19

(54) **Verfahren zum Leimen von Papier mit anionischen, hydrophoben Leimungsmitteln und kationischen Retentionsmitteln.**

(30) Priorität: **06.08.84 CH 3770/84**

(43) Veröffentlichungstag der Anmeldung: **19.02.86 Patentblatt 86/8**

(45) Bekanntmachung des Hinweises auf die Patenterteilung: **14.09.88 Patentblatt 88/37**

(84) Benannte Vertragsstaaten: **AT BE CH DE FR GB IT LI NL SE**

(56) Entgegenhaltungen:
**EP - A - 0 096 654**
**DE - B - 1 276 049**
**GB - A - 723 869**
**US - A - 3 303 211**
**US - A - 3 488 380**
**US - A - 4 295 931**

**J.P. CASEY: "Pulp and paper chemistry and chemical technology", 3. Auflage, Band III, 1981, Seiten 1577-1583,1588-1592, John Wiley & Sons Inc., New York, US;**

(73) Patentinhaber: **CIBA-GEIGY AG, Klybeckstrasse 141, CH-4002 Basel (CH)**

(72) Erfinder: **Bernheim, Michael, Dr., Stadtjägerstrasse 7, D-8900 Augsburg (DE)**
Erfinder: **Strasilla, Dieter, Dr., Efringerstrasse 26, D-7858 Weil am Rhein (DE)**
Erfinder: **De Sousa, Bernardo, Dr., Paradiesstrasse 15, CH-4125 Riehen (CH)**
Erfinder: **Rohringer, Peter, Sechsjuchartenstrasse 1, CH-4124 Schönenbuch (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Der vorliegenden Erfindung liegt die Aufgabe zugrunde, dem Papierhersteller leicht zugängliche und auf einfache Art erhältliche Leimungsmittel zur Verfügung zu stellen, die unter Mitverwendung von herkömmlichen, kationischen Retentionsmitteln in neuartiger Kombination geeignet sind, eine gute Leimung sowohl bei der Papierherstellung aus Faserstoffsuspensionen (Massenleimung) als auch bei der Herstellung von Papier mit geleimter Oberfläche (Oberflächenleimung) zu bewirken.

Diese Aufgabe wird erfindungsgemäss so gelöst, dass bei der Papierherstellung unter Mitverwendung von polymeren, kationischen Retentionsmitteln, aromatische Verbindungen als Leimungsmittel eingesetzt werden, die einen einzigen, langkettigen, hydrophoben Substituenten und mindestens eine anionische, acide (saure), d.h. freie oder in Salzform vorliegende Gruppe aufweisen, wobei der hydrophobe Substituent am aromatischen Kern über ein Brückenglied verbunden ist, das mindestens 1 Kohlenstoffatom und mindestens 2 Heteroatome in der Hauptkette enthalt.

Gegenstand der vorliegenden Erfindung ist somit ein Verfahren zum Leimen von Papier oder Karton, d.h. ein Verfahren zur Herstellung von in der Masse geleimtem Papier oder Karton oder von Papier mit geleimter Oberfläche, das dadurch gekennzeichnet ist, dass man mindestens

(A) ein Leimungsmittel, das aus einer aromatischen Verbindung besteht, die am aromatischen Kern einen einzigen, hydrophoben Substituenten mit mindestens 5 Kohlenstoffatomen und mindestens eine anionische, freie oder in Salzform vorliegende Gruppe aufweist, wobei der hydrophobe Substituent mit dem aromatischen Kern über ein Ester-, Amid-, Urethan- oder Harnstoffbrückenglied verknüpft, bei Ester- und Amidbrückengliedern die CO-Einheit an den hydrophoben Rest gebunden und zwischen den Brückengliedern und dem aromatischen Kern gegebenenfalls eine Methylengruppe vorhanden ist, und

(B) ein polymeres, kationisches Retentionsmittel einsetzt. Im erfindungsgemässen Verfahren werden bei der Massenleimung von Papier oder Karton die Komponenten (A) und (B) zu wässrigen, cellulosehaltigen, gegebenenfalls füllmittelhaltigen Faserstoffsuspensionen in beliebiger Reihenfolge oder gleichzeitig gegeben während bei der Papieroberflächenleimung das Papier mit einer wässrigen Leimflotte, welche die Komponenten (A) und (B) enthält, imprägniert und getrocknet wird.

Weitere Erfindungsgegenstände bilden

– die wässrigen Zusammensetzungen zur Durchführung des Papierleimungsverfahrens, die, sofern das Leimungsmittel (A) und das Retentionsmittel (B) bei der Massenleimung in beliebiger Reihenfolge zur Faserstoffsuspension separat gegeben werden, neben fakultativen üblichen Zusätzen das Leimungsmittel (A) allein, das mindestens teilweise in Form von Salzen vorliegt, oder, sofern das Leimungsmittel (A) und das Retentionsmittel (B) bei der Massenleimung zur Faserstoffsuspension gleichzeitig gegeben werden, oder als Leimflotte zur Oberflächenleimung des Papiers eingesetzt werden, sowohl das gegebenenfalls mindestens teilweise in Salzform vorliegende Leimungsmittel (A) als auch das Retentionsmittel (B) neben fakultativen, üblichen Zusätzen enthalten,

– nach dem erfindungsgemässen Verfahren geleimtes Papier oder geleimter Karton und

– die Verwendung des Leimungsmittels (A) der angegebenen Art zum Leimen von Papier oder Karton.

Teilweise stellen die angegebenen Leimungsmittel (A) neue Verbindungen dar, welche zusammen mit dem Verfahren zu deren Herstellung ebenfalls weitere Gegenstände der vorliegenden Erfindung bilden.

Als wesentliches Merkmal weisen die erfindungsgemässen Leimungsmittel (A) im allgemeinen 1 oder 2 anionische Gruppen auf, die in der Regel als acide, am aromatischen Kern gebundene Carboxyl-, Hydroxyl- oder Sulfogruppen vorliegen. Sofern zwei acide Gruppen vorhanden sind, ist eine dieser beiden Gruppen vorzugsweise eine Hydroxylgruppe. Leimungsmittel, die nur eine solche acide Gruppe aufweisen, sind bevorzugt. Falls solche Gruppen als Salze, z.B. als Amin-, Ammonium- oder Natriumsalze vorliegen, können sie in wässrigem Medium bei den üblicherweise bei der Papierherstellung vorliegenden pH-Werten der Faserstoffsuspensionen Anionen bilden. Unter den genannten Bedingungen können andererseits die kationischen Retentionsmittel (B) Kationen bilden. Die Fähigkeit der Leimungsmittel und der Retentionsmittel, bei den Bedingungen der Papierherstellung Anionen bzw. Kationen zu bilden, kann auch als anionaktiv bzw. kationaktiv bezeichnet werden. Somit können die anionischen Leimungsmittel und die kationischen Retentionsmittel auch anionaktive Leimungsmittel bzw. kationaktive Retentionsmittel genannt werden.

Als weiteres kennzeichnendes Merkmal weisen die Leimungsmittel (A) einen einzigen, vorzugsweise aliphatischen, hydrophoben Substituenten mit mindestens 5, vor allem 5 bis 22, vorzugsweise 11 bis 22, insbesondere 16 bis 20 Kohlenstoffatome auf. Bevorzugte hydrophobe Substituenten sind Alkenyl- oder vor allem Alkylreste, welche sich in der Regel von ungesättigten oder vor allem gesättigten Fettsäuren oder sogenannten Fettisocyanaten ableiten. Die hydrophoben Substituenten bestehen somit nur aus Kohlenstoff- und Wasserstoffatomen und sind an einem zweiwertigen Brückenglied der angegebenen Art gebunden.

Als Fettsäuren, aus welchen sich die genannten hydrophoben Substituenten ableiten, seien ungesättigte oder vor allem gesättigte $C_6$–$C_{22}$- vorzugsweise $C_{11}$–$C_{22}$-, insbesondere $C_{16}$–$C_{20}$-Fettsäuren erwähnt. Bei diesen handelt es sich z.B. um Capron-, vorzugsweise Capryl-, Caprin-, Laurin-, Myristin- oder Myristolein-, Palmitolein-,

Elaeostearin-, Clupanodonsäure, insbesondere Öl-, Elaidin-, Eruka-, Linol- und Linolensäure. Hierbei kommt der Palmitin-, Stearin-, Öl- und Behensäure eine besondere Bedeutung zu, wobei Palmitin- und vor allem Stearinsäure im Vordergrund des Interesses stehen. Auch gut zugängliche, technische Gemische dieser Fettsäuren kommen in Betracht. Synthetische Fettsäuren, die z. B. durch Oxosynthese herstellbar sind, werden von der angegebenen Definition auch umfasst.

Die sogenannten Fettisocyanate weisen einen langkettigen, sich in der Regel von einer Fettsäure der angegebenen Art ableitenden Rest auf. Diese Fettisocyanate sind aus den entsprechenden Fettaminen und Phosgen erhältlich. Seiner guten Zugänglichkeit wegen kommt dem Octadecylisocyanat, auch Stearylisocyanat genannt, eine besondere Bedeutung zu. Auch technische Gemische der Fettisocyanate der angegebenen Art kommen in Frage.

Die Verknüpfungsart, mit welcher der hydrophobe Substituent an dem aromatischen Kern verbunden ist, bildet ein weiteres Kennzeichen der Leimungsmittel (A). Diese zweiwertigen Brückenglieder stellen nämlich, wie vorstehend erwähnt, Esterbrücken –O–CO–, Amidbrücken –NH–CO–, Urethanbrücken –NH–CO–O– oder Harnstoffbrücken –NH–CO–NH– dar, die ein Sauerstoffatom, ein Stickstoffatom, ein Sauerstoff- und ein Stickstoffatom oder zwei Stickstoffatome in der Hauptkette aufweisen, wobei die endständige Carbonyleinheit –CO– der Ester- und Amidbrückenglieder (im Gegensatz zur Carbonyleinheit der Urethan- oder Harnstoffbrückenglieder, die nicht endständig ist) stets an dem hydrophoben Rest, während das Sauerstoffatom der Esterbrücke und die NH-Einheit der Amidbrücke stets am aromatischen Kern der als Leimungsmittel verwendeten, aromatischen Verbindung verknüpft sind. Zwischen den vorstehend beschrieb enen Brückengliedern, vor allem dem Ester- und insbesondere dem Amidbrückenglied und dem aromatischen Kern kann zusätzlich eine Methylengruppe gegebenenfalls vorhanden sein. Bevorzugt sind die Brückenglieder jedoch direkt mit dem aromatischen Kern verknüpft.

Als Kern der aromatischen Verbindungen kommen z.B. Tetrahydro- oder Dihydronaphthylen, vor allem Naphthylen und insbesondere Phenylen in Betracht. Hierbei sind diese zweiwertigen aromatischen Reste durch z.B. Amino, Nitro und/oder Halogen, vorzugsweise Brom oder vor allem Chlor, gegebenenfalls substituiert. Die unsubstituierten zweiwertigen, aromatischen Reste sind jedoch bevorzugt.

Demgemäss setzt man als Komponente (A) des erfindungsgemässen Papierleimungsverfahrens Leimungsmittel der Formel

$$\begin{array}{l}(A_2)_{m-1}\\ \;\;|\\ A_1-D_1-(CH_2)_{n-1}-X_1-R_1\end{array} \qquad (1)$$

ein, worin

$A_1$ und $A_2$ unabhängig voneinander jeweils eine anionische, in Salzform vorliegende oder acide Carboxyl-, Hydroxyl- oder Sulfogruppe,

$D_1$ durch Halogen, Nitro, Amino oder Hydroxyl substituiertes oder unsubstituiertes Phenylen, Naphthylen, Dihydronaphthylen oder Tetrahydronaphthylen,

$R_1$ Alkyl oder Alkenyl mit 5 bis 22 Kohlenstoffatomen,

$X_1$ ein Brückenglied der Formel –O–CO–, –NH–CO–, –NH–CO–O–, –O–CO–NH–, –NH–CO–NH–, wobei endständige –CO-Einheiten des Brückengliedes an den Alkyl- oder Alkenylrest $R_1$ geknüpft sind, und

m und n unabhängig voneinander jeweils 1 oder 2 bedeuten.

Sofern $X_1$ ein Urethanbrückenglied bedeutet, ist dessen endständige NH-Einheit wegen besserer Zugänglichkeit der entsprechenden Verbindungen vorzugsweise an den Alkyl- oder Alkenylrest $R_1$ geknüpft.

Da die Methylengruppe vor allem in Anwesenheit eines Ester- oder Amidbrückengliedes vorhanden ist, als aromatischer Kern vor allem gegebenenfalls substituiertes Naphthylen oder insbesondere gegebenenfalls substituiertes Phenylen in Betracht kommt und die methylengruppenhaltigen Verbindungen vorzugsweise nur eine einzige anionische Gruppe der angegebenen Art aufweisen, werden als weiter bevorzugte Leimungsmittel solche der Formel

$$A_1-D_2-(CH_2)_{n-1}-X_2-CO-R_2 \qquad (2)$$

oder

$$A_1-D_2-X_2-CO-NH-R_3 \qquad (3)$$

eingesetzt, worin

$D_2$ durch Chlor, Brom, Nitro, Amino oder Hydroxyl substituiertes oder unsubstituiertes Phenylen oder Naphthylen,

$R_2$ Alkyl oder Alkenyl mit 5 bis 21 Kohlenstoffatomen,

$R_3$ Alkyl oder Alkenyl mit 6 bis 22 Kohlenstoffatomen,

$X_2$ –O– oder –NH– und

n 1 oder 2 bedeuten und

$A_1$ die angegebenen Bedeutungen hat.

Hierbei sei darauf hingewiesen, dass der Rest $-CO-R_2$ in Formel (2) sich von $C_6$–$C_{22}$-Fettsäuren ableitet, worin die Carbonyleinheit der Fettsäure inbegriffen ist. Darum bedeutet $R_2$ Alkyl oder Alkenyl mit 5 bis 21 Kohlenstoffatomen im Gegensatz zu $R_3$ in Formel (3), das 6 bis 22 Kohlenstoffatome aufweist und sich von einem $C_6$–$C_{22}$-Fettisocyanat ableitet.

Die meist bevorzugten Vertreter von methylengruppenhaltigen Leimungsmitteln sind am aromatischen Kern substituiert und weisen Amidbrückenglieder und hydrophobe Substituenten auf, die sich vorzugsweise von $C_{16}$–$C_{22}$-Fettsäuren ableiten. Als Leimungsmittel dieser Art, werden somit vorzugsweise solche der Formel

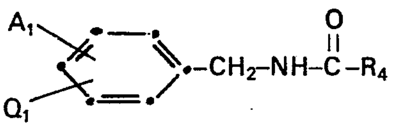 (4)

eingesetzt, worin $Q_1$ Chlor, Brom, Nitro oder Amino, $R_4$ Alkyl oder Alkenyl mit 15 bis 21 Kohlenstoffatomen und $A_1$ die angegebenen Bedeutungen hat.

Bevorzugt weisen jedoch die Leimungsmittel keine solchen Methylengruppen auf und sind am aromatischen Kern nicht substituiert. Zudem weisen sie eine anionische bzw. acide Gruppe der angegebenen Art und gegebenenfalls eine Hydroxylgruppe als zweite anionische bzw. acide Gruppe auf. Als Vertreter von Leimungsmitteln dieser Art, welche Amid- oder Esterbrückenglieder und hydrophobe Substituenten aufweisen, die sich vorzugsweise von $C_{16}$–$C_{22}$-Fettsäuren ableiten, werden z.B. solche der Formel

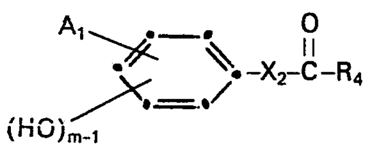 (5)

oder

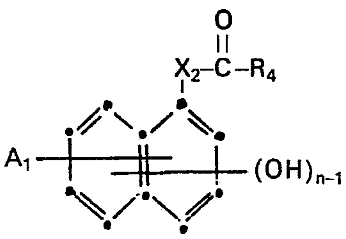 (6)

eingesetzt, worin m 1 oder 2 ist und $A_1$, $R_4$ und $X_2$ die angegebenen Bedeutungen haben.

Falls hingegen die bevorzugten methylengruppenfreien Leimungsmittel Urethan- oder Harnstoffbrückenglieder und hydrophobe Substituenten aufweisen, die sich vorzugsweise von $C_{16}$–$C_{22}$-Fettaminen ableiten, wobei der aromatische Kern unsubstituiert ist, werden z.B. solche der Formel

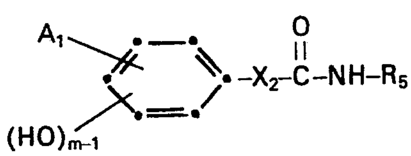 (7)

oder

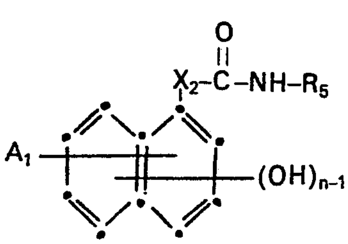 (8)

eingesetzt, worin $R_5$ Alkyl oder Alkenyl mit 16 bis 22 Kohlenstoffatomen bedeutet und $A_1$, $R_5$, $X_2$ und m die angegebenen Bedeutungen haben. Hierbei sind die Leimungsmittel mit Phenylenresten der Formel (5) oder (7) gegenüber den Leimungsmitteln mit Naphthylenresten der Formel (6) oder (8) bevorzugt.

Gegenüber den Leimungsmitteln der Formeln (6) und (8) sind solche der Formel (4), vor allem der Formel (7) und insbesondere der Formel (5) bevorzugt.

Als spezifische Vertreter der Leimungsmittel der Formel (4) kommen z.B. solche der Formel

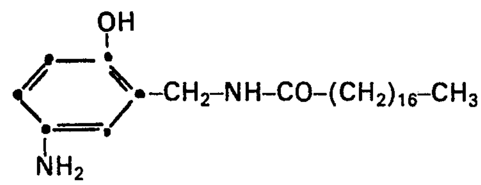 (9)

und

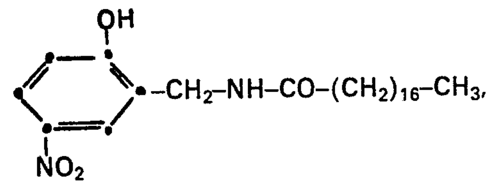 (10)

als spezifische Vertreter der Leimungsmittel der Formel (5) z.B. solche der Formel

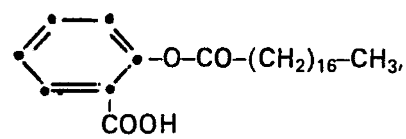 (11)

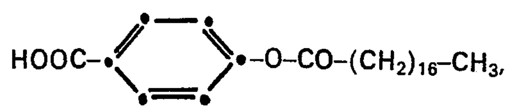 (12)

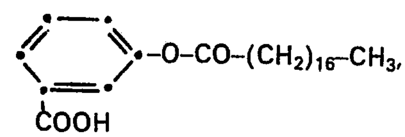 (13)

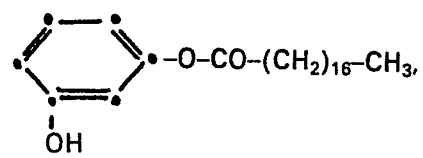 (14)

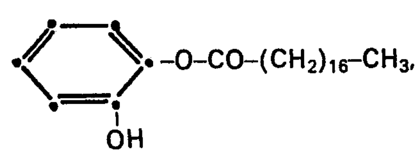 (15)

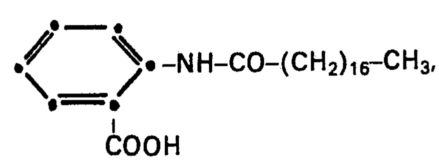 (16)

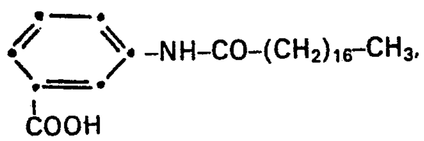 (17)

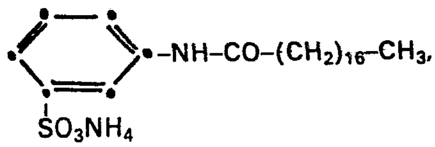 (18)

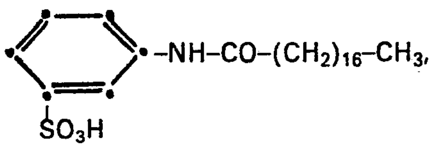 (19)

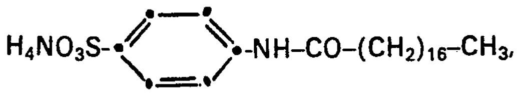 (20)

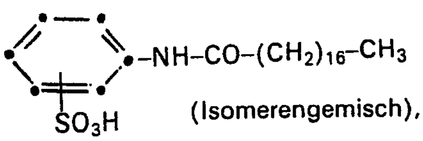 (21)

(Isomerengemisch),

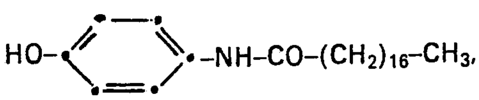 (22)

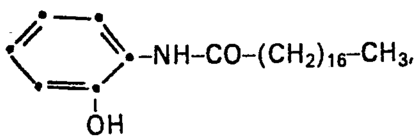 (23)

und

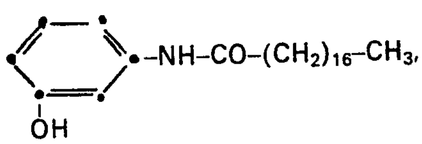 (24)

als spezifischer Vertreter der Formel (6) z.B. das Leimungsmittel der Formel

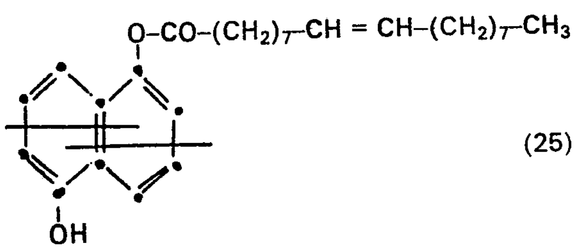 (25)

als spezifische Vertreter der Leimungsmittel der Formel (7) z.B. solche der Formel

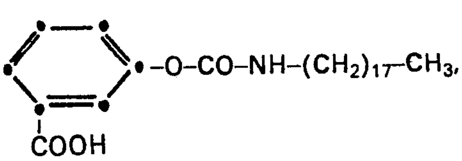 (26)

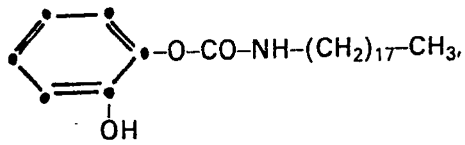 (27)

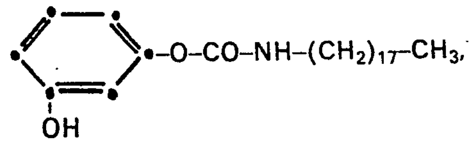 (28)

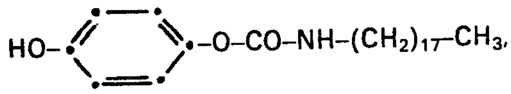 (29)

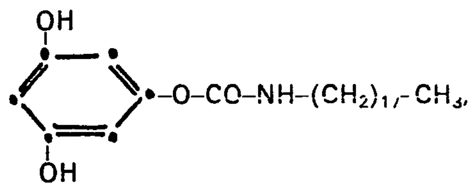 (30)

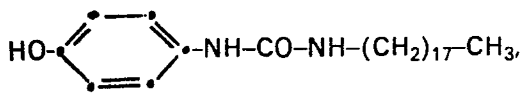 (31)

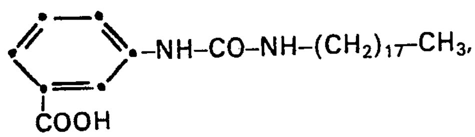 (32)

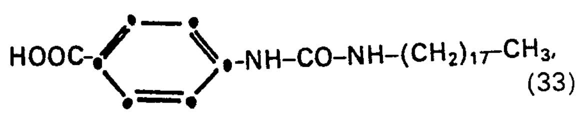 (33)

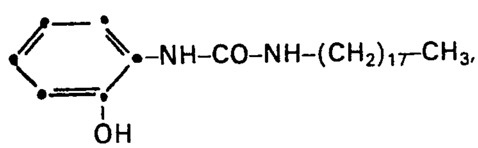 (34)

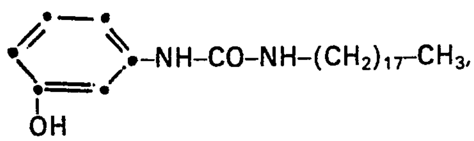 (35)

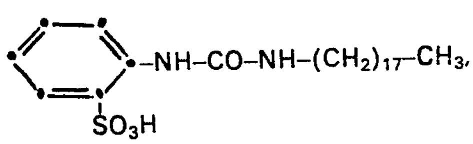 (36)

und

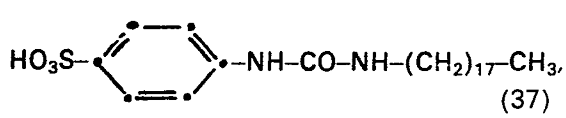 (37)

und als spezifischer Vertreter der Formel (8) z.B. das Leimungsmittel der Formel

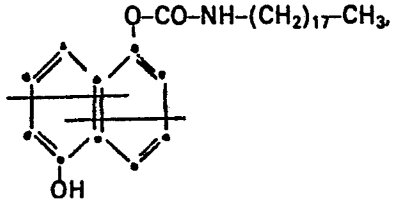

(38)

in Betracht.

Hierbei stehen die Leimungsmittel der Formel (23) und vor allem der Formel (15) im Vordergrund des Interesses.

Vor ihrem Einsatz als Komponente (A) im erfindungsgemässen Papierleimungsverfahren brauchen die Leimungsmittel nach erfolgter Herstellung im allgemeinen nicht durch z.B. Umkristallisieren gereinigt zu werden, sondern können in der Regel direkt, d.h. als Rohprodukte verwendet werden.

Vor allem bei separater Zugabe (in beliebiger Reihenfolge) des Leimungsmittels (A) und des Retentionsmittels (B) zur Faserstoffsuspension beim erfindungsgemässen Verfahren zum Leimen von Papier oder Karton in der Masse ist es zweckmässig, das Leimungsmittel mindestens teilweise in Salzform einzusetzen. Solche Salze können bei Bedarf dadurch erhalten werden, dass man die Leimungsmittel (A) nach erfolgter Herstellung durch Zugabe u.a. eines Alkylamins oder Alkanolamins mit insgesamt höchstens 6 Kohlenstoffatomen, z.B. Trimethylamin, Triethylamin, Monoethanolamin, Diethanolamin, vor allem durch Zugabe von Ammoniak oder eines Alkalimetallhydroxides, beispielsweise Kalium- oder vor allem Natriumhydroxid, in der Regel in wässrigem Medium bei Raumtemperatur (etwa 15 bis etwa 25 °C) in die entsprechenden Salze ganz oder teilweise überführt. Zweckmässigerweise wird ein Alkalimetallhydroxid, z.B. Kalium- oder vor allem Natriumhydroxid oder insbesondere Ammoniak in der Regel in Form ihrer verdünnten, etwa 1 bis 10 gewichtsprozentigen, wässrigen Lösungen verwendet. Zweckmässig wird in der Regel höchstens 2 Mol, vor allem 0,1 bis 1,5, insbesondere 0,9 bis 1,1 Mol Ammoniak oder Alkalihydroxid pro vorhandene acide Gruppe des Leimungsmittels eingesetzt. Die als Salze vorliegenden Leimungsmittel weisen somit z.B. acide Carboxyl-, Hydroxyl- oder Sulfogruppen auf, die mindestens teilweise in die Gruppen $-COO^{\ominus}M^{\oplus}$, $-O^{\ominus}M^{\oplus}$ oder $SO_3^{\ominus}M^{\oplus}$ überführt werden, worin $M^{\oplus}$ die entsprechenden Amin-, Ammonium- oder Alkalimetall-Kationen bedeutet.

Bevorzugte Leimungsmittel (A) der angegebenen Art weisen Molekulargewichte von etwa 200 bis etwa 700, vorzugsweise etwa 350 bis etwa 600 und infolge ihres Gehaltes an mindestens einer aciden Gruppe der angegebenen Art eine Säurezahl (mg KOH/g Substanz) von etwa 80 bis etwa 500, vorzugsweise etwa 100 bis etwa 300 auf.

Wie bereits angegeben, stellen die im erfindungsgemässen Papierleimungsverfahren als Komponente (A) eingesetzten Leimungsmittel zum Teil an sich bekannte und zum Teil an sich neue Verbindungen dar, die nach an sich bekannten Methoden hergestellt werden.

So stellen z.B. Leimungsmittel, die zwischen einem substituierten Phenylenring und dem Amidbrückenglied Methylengruppen aufweisen, an sich neue Verbindungen dar, die u.a. der Formel

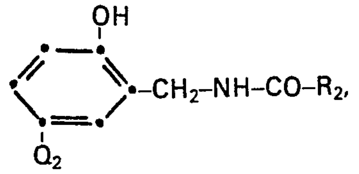

(39)

oder deren Salze, z.B. deren Ammonium-, Amin- oder Natriumsalze, worin $Q_2$ Nitro oder Amino und $R_2$ Alkyl oder Alkenyl mit 5 bis 21, vorzugsweise 15 bis 21 Kohlenstoffatome bedeuten, und insbesondere der Formel (9) oder (10) entsprechen, wobei sich das Verfahren zur Herstellung der Verbindungen der Formel (39) dadurch auszeichnet, dass man ein methyloliertes Fettsäureamid der Formel

$$R_2-CO-NH-CH_2OH, \qquad (40)$$

worin $R_2$ die angegebenen Bedeutungen hat, mit p-Amino- oder p-Nitrophenol auf an sich bekannte Weise, gegebenenfalls mit einem Überschuss an p-Amino- oder p-Nitrophenol, jedoch vorzugsweise in etwa äquimolaren Mengen umsetzt.

Die US Patentschrift 2 448 247 u.a. offenbart als wasserabweisendes Mittel für Textilien o- und p-Stearoyloxybenzoesäure, d.h. Verbindungen der Formel (5), worin $A_1$ für Carboxyl in o- oder p-Stellung, $R_4$ für $C_{17}$-Alkyl, $X_2$ für –O– und m für 1 stehen, sowie 3-Stearoyloxy- oder 3-Lauroyloxynaphthalin-2-monocarbonsäure, d.h. Verbindungen der Formel (6), worin $A_1$ für Carboxyl, $R_4$ für $C_{17}$- oder $C_{11}$-Alkyl, $X_2$ für –O– und m für 1 stehen, wobei $A_1$ in 2-Stellung und $-X_2-CO-R_4$ in 3-Stellung liegen. Diese Referenz macht jedoch keinerlei Angaben über die entsprechenden m- und 1,5-Isomere. Ferner offenbart z.B. die europäische Patentanmeldung 60 092 als Aktivsubstanz von kosmetischen Zusammensetzungen zur Depigmentierung der Haut Verbindungen der Formel (5), worin $A_1$ für Hydroxyl in p-Stellung, $R_4$ für $C_1$–$C_{20}$-Alkyl oder -Alkenyl, $X_2$ für –O– und m für 1 stehen, insbesondere Hydrochinonmonostearat. Diese Referenz macht keinerlei Angaben über entsprechende Isomere, d.h. die m- und o-Isomere. Schliesslich beschreibt z.B. die Deutsche Offenlegungsschrift 2 260 703 als Färbereihilfsmittel anionaktive Verbindungen der Formel (5) oder (6), worin $A_1$ in beliebiger Stellung für $SO_3Na$ oder $SO_3NH_4$, $R_4$ für $C_7$–$C_{21}$-Alkyl oder -Alkenyl, $X_2$ für –O– und m für 2 oder vorzugsweise 1 stehen. Somit sind die Verbindungen an sich neu, die u.a. der Formel

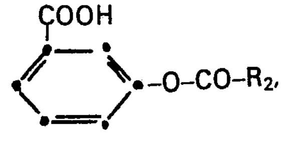

(41)

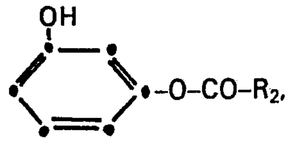

(42)

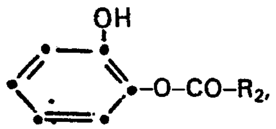

(43)

oder

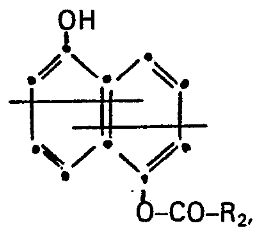

(44)

oder deren Salze, worin $R_2$ Alkyl oder Alkenyl mit 5 bis 21, vorzugsweise 15 bis 21 Kohlenstoffatomen bedeuten, und insbesondere der Formeln (13), (14), (15) oder (25) entsprechen, wobei sich das Verfahren zu deren Herstellung dadurch auszeichnet, dass man eine Fettsäure der Formel

$R_2$–COOH (45)

oder deren Halogenid, insbesondere deren Chlorid, worin $R_2$ die angegebenen Bedeutungen hat, mit 3-Hydroxybenzoesäure, Resorcin, Brenzkatechin oder 1,5-Dihydroxynaphthalin auf an sich bekannte Weise, gegebenenfalls mit einem Überschuss der genannten aromatischen Ausgangskomponenten, vor allem einem Überschuss an Resorcin, Brenzkatechin oder 1,5-Dihydroxynaphthalin, jedoch vorzugsweise in etwa äquimolaren Mengen umsetzt.

Die US-Patentschrift 3 773 663 u.a. offenbart als Beschichtungsmittel für Asbest in Schmierfetten Verbindungen der Formel (5), worin $A_1$ in beliebiger Stellung für ein Alkalimetallcarboxylat, $R_4$ für $C_{10}$–$C_{30}$-Alkyl oder -Alkenyl, $X_2$ für –NH– und m für 1 stehen. Ferner offenbart z.B. die deutsche Auslegeschrift 1 140 077 als Haftmittel für Emulsions- und Hilfsschichten auf Acetylcellulosefilmen Verbindungen der Formel (5), worin $A_1$ in beliebiger Stellung für eine Sulfogruppe, $R_4$ für Alkyl mit mindestens 6 Kohlenstoffatomen, $X_2$ für –NH– und m für 1 stehen. Schliesslich offenbart z.B. die US-Patentschrift 4 002 701 N-Alkanoylp-aminophenol als Stabilisatoren in Polyethylen- und -vinylharzen, d.h. Verbindungen der Formel (5), worin $A_1$ in p-Stellung für Hydroxyl, $R_4$ $C_8$–$C_{23}$-Alkyl, $X_2$ für –NH– und m für 1 stehen. Diese Referenz macht jedoch keinerlei Angaben über die o- und m-Isomere, z.B. N-Alkanoyl- o- oder -m-Aminophenol. Dies gilt auch für die US-Patentschrift 4 320 209, die N-Stearoyl-p-aminophenol als Mittel zur Beschleunigung der Kristallisation von Polymeren offenbart, für z.B. die US-Patentschrift 3 288 885, die N-$C_2$–$C_{20}$-Acyl-p-aminophenol als Stabilisatoren in Polymerzusammensetzungen offenbart und für die französische Patentschrift 2 115 676, die N-Stearoyl- oder N-Lauroyl-p-aminophenol als Zusatz in Polyamidformmassen offenbart.

Somit sind die Verbindungen an sich neu, die u.a. der Formel

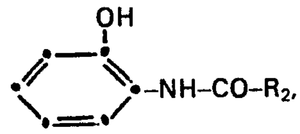

(46)

oder

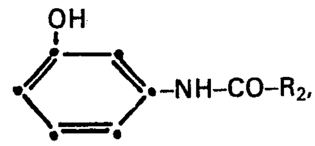

(47)

oder deren Salze, worin $R_2$ Alkyl oder Alkenyl mit 5 bis 21, vorzugsweise 15 bis 21 Kohlenstoffatomen bedeutet, und insbesondere der Formel (23) oder (24) entsprechen, wobei sich das Verfahren zu deren Herstellung dadurch auszeichnet, dass man eine Fettsäure der Formel (45) oder deren Halogenid, insbesondere Chlorid, mit o- oder m-Aminophenol auf an sich bekannte Weise, gegebenenfalls mit einem Überschuss an o- oder m-Aminophenol, jedoch vorzugsweise in etwa äquimolaren Mengen umsetzt.

Die Urethanbrückenglieder aufweisenden Leimungsmittel stellen im allgemeinen auch an sich neue Verbindungen dar, die vorzugsweise der Formel (7) oder deren Salzen entsprechen, worin $X_2$ für –O– steht. Dies trifft vor allem für die Verbindungen der Formel

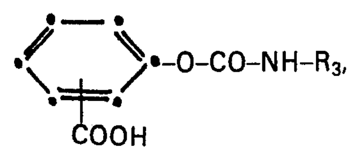

(48)

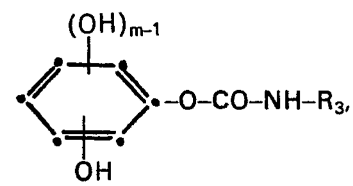

(49)

oder

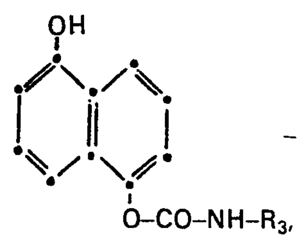

(50)

oder deren Salze, worin $R_3$ Alkyl oder Alkenyl mit

6 bis 22, vorzugsweise 16 bis 22 Kohlenstoffatomen und m 1 oder 2 bedeuten, und insbesondere für die Verbindungen einer der Formeln (26) bis (30) oder (38) zu, wobei sich das Verfahren zu deren Herstellung dadurch auszeichnet, dass man ein Alkyl- oder Alkenylisocyanat der Formel

$R_3$–N=C=O, (51)

worin $R_3$ die angegebenen Bedeutungen hat, mit 2-, 3- oder 4-Hydroxybenzoesäure, Brenzkatechin, Resorcin, Hydrochinon, Phloroglucin, Hydroxyhydrochinon, Pyrogallol oder 1,5-Dihydroxynaphthalin auf an sich bekannte Weise, gegebenenfalls mit einem Überschuss der genannten aromatischen Ausgangskomponenten, vor allem solcher, die 2 oder 3 Hydroxylgruppen aufweisen, jedoch vorzugsweise in etwa äquimolaren Mengen umsetzt. Hierbei ist das Isocyanat der Formel (51) im allgemeinen aus den entsprechenden Fettaminen und Phosgen herstellbar und im Handel erhältlich.

Die US-Patentschrift 2 683 083 u.a. offenbart als Antioxidantien für Benzin Urethanbrückenglieder aufweisende Verbindungen der Formel (7), worin $A_1$ für Hydroxyl in p-Stellung, $R_2$ für z.B. n-Octadecyl, $X_2$ für –NH– und m für 1 stehen, macht jedoch keinerlei Angaben über die entsprechenden o- und m-Isomere.

Somit sind Verbindungen an sich neu, die u.a. der Formel

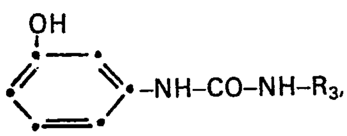 (52)

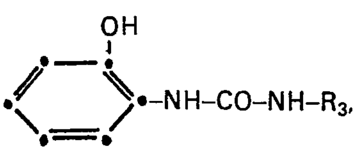 (53)

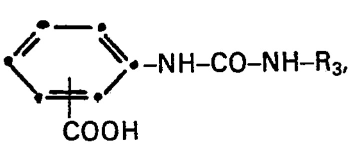 (54)

oder

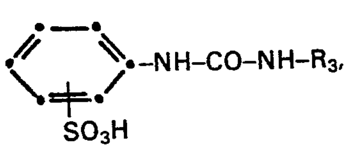 (55)

oder deren Salze, worin $R_3$ Alkyl oder Alkenyl mit 6 bis 22, vorzugsweise 16 bis 22 Kohlenstoffatomen bedeuten, und insbesondere einer der Formeln (32) bis (37) entsprechen, wobei sich das Verfahren zu deren Herstellung dadurch auszeichnet, dass man ein Alkyl- oder Alkenylisocyanat der Formel (51) mit 2- oder 3-Aminophenol, 2-, 3- oder 4-Aminobenzoesäure oder Anilin-2-, -3- oder -4-Sulfonsäure auf an sich bekannte Weise, gegebenenfalls mit einem Überschuss der genannten, aromatischen Ausgangskomponenten, jedoch vorzugsweise in etwa äquimolaren Mengen umsetzt.

Im erfindungsgemässen Papierleimungsverfahren wird neben dem neuen, vorstehend beschriebenen, anionischen oder aciden Leimungsmittel (A) stets ein polymeres, kationisches Retentionsmittel (B) eingesetzt, welches in der Regel ein Molekulargewicht von mindestens etwa 1000, vorzugsweise etwa 2000 bis etwa 2 000 000 aufweist. Retentionsmittel mit Molekulargewichten im Bereich von 10 000 bis 100 000 sind besonders bevorzugt. Grundsätzlich kommt jedes handelsübliche Retentionsmittel in Betracht für seinen Einsatz im erfindungsgemässen Verfahren. Als Beispiele herkömmlicher Retentionsmittel (B), die sich besonders gut dazu eignen, zusammen mit dem Leimungsmittel (A) im erfindungsgemässen Papierleimungsverfahren eingesetzt zu werden, seien Polyalkylenimine Epihalogenhydrin-Addukte von Umsetzungsprodukten aus Polyalkylenpolyaminen und aliphatischen Dicarbonsäuren; Epihalogenhydrin-Addukte von Umsetzungsprodukten aus Polyalkylenpolyaminen, Dicyandiamid und gegebenenfalls unveresterten oder mit Alkanolen veresterten, organischen Dicarbonsäuren; Umsetzungsprodukte aus Dicyandiamid, Formaldehyd, Ammoniumsalzen starker anorganischer Säuren und Alkylendiaminen oder Polyalkylenpolyaminen; kationisch modifizierte Stärken oder Kohlenhydrate aus Johannisbrot- oder Guarkernmehl; Copolymerisate auf Basis von Polyamid-Aminen und Umsetzungsprodukte aus Epihalogenhydrinen und polymerisierten Diallylaminen erwähnt.

Bevorzugte Epichlorhydrin-Addukte von Umsetzungsprodukten aus Polyalkylenpolyaminen und aliphatischen Dicarbonsäuren sind z.B. in der britischen Patentschrift 865 727, Epichlorhydrin-Addukte aus Umsetzungsprodukten aus Dicyandiamid und Diethylentriamin oder Triethylentetramin z.B. in der deutschen Offenlegungsschrift 2 710 061 und in der britischen Patentschrift 1 125 486, Epichlorhydrin-Addukte von Umsetzungsprodukten aus Diethylentriamin, Dicyandiamid und unveresterten oder vorzugsweise mit Niederalkanolen veresterten Dicarbonsäuren, insbesondere Dimethyladipat, z.B. in der britischen Patentschrift 1 125 486 und Umsetzungsprodukte aus Dicyandiamid, Formaldehyd, Ammoniumsalzen starker anorganischer Säuren und aus Ethylendiamin oder Triethylentetramin, z.B. in der US-Patentschrift 3 491 064 beschrieben. Bevorzugte kationisch modifizierte Stärken oder Kohlehydrate aus Johannisbrot- oder Guarkernmehl sind z.B. Alkylenoxid-Addukte dieser Stärken oder Kohlenhydrate, wobei das eingesetzte Alkylenoxid 2 oder 3 Kohlenstoffatome im Alkylenrest und quaternäre Ammoniumgruppen aufweist oder insbesondere z.B. ein Trimethylglycidylammoniumhalogenid darstellt. Copolymerisate auf Basis von Polyamid-Aminen weisen Molekulargewichte von $10^3$ bis $10^5$, vorzugsweise $10^3$ bis $10^4$ auf und sind z.B. aus aliphatischen, gesättigten Dicarbonsäuren mit 2 bis 10, vorzugsweise 3 bis 6 Kohlenstoffatomen, insbesondere Adipinsäure, und Polyalkylenpolyaminen, z.B. Polypropylen- und Polyethylenpolyaminen, insbesondere Dimethylaminohydroxypropyldiethylentriamin, erhältlich. Sie sind z.B. in CTFA Cosmetic Ingredient Dictionary, 3. Auflage 1982, der Cosmetic Toiletry und Fragrance Association beschrieben. Umsetzungsprodukte aus Epihalogenhydrinen und polymerisierten Diallylaminen weisen bevorzugt Molekulargewichte von 1000 bis 2000 auf und sind z.B. in den US-Patentschriften 3 700 623 und 4 279 794 beschrieben.

Als Retentionsmittel (B), die zur Verwendung zusammen mit den Leimungsmitteln (A) im erfindungsgemässen Papierleimungsverfahren im Vordergrund des Interesses stehen, sei eine mit einem quaternäre Ammoniumgruppen enthaltenden Propylenoxid modifizierte Mais- oder Kartoffelstäre, deren 25%ige Anschlämmung in destilliertem Wasser bei 20 °C einen pH-Wert von 4,2 bis 4,6 aufweist, ein Polyethylenimin, das ein Molekulargewicht von 10 000 bis 100 000 aufweist, ein Epichlorhydrin-Addukt eines Umsetzungsproduktes aus Triethylentetramin und Dicyandiamid, ein Epichlorhydrin-Addukt eines Umsetzungsproduktes aus Diethylentriamin, Dicyandiamid und Dimethyladipat, ein Umsetzungsprodukt aus Dicyandiamid, Formaldehyd, Ammoniumchlorid und Ethylendiamin, ein Epichlorhydrin-Addukt eines Poly-N-methyldiallylamins und ein Copolymerisat aus Adipinsäure und Dimethylaminohydroxypropyl-diethylentriamin genannt.

Verfahrensgemäss werden bei der Massenleimung von Papier oder Karton in der Regel 0,02 bis 3, vorzugsweise 0,05 bis 3, insbesondere 0,1 bis 0,8 Gewichtsprozent des Leimungsmittels (A) und 0,02 bis 3, vorzugsweise 0,05 bis 3, insbesondere 0,1 bis 0,4 Gewichtsprozent des Retentionsmittels (B), bezogen jeweils auf Trockensubstanz an (A) und (B) und auf den Feststoffgehalt der Faserstoffsuspension, eingesetzt. 0,02 bis etwa 0,05 Gewichtsprozent des Leimungsmittels (A) und des Retentionsmittels (B) reichen nur für das sogenannte «size press control», das mit konventionellen Leimungstests nicht erfassbar ist (vgl. z.B. Artikel «Control and Understanding of Size Press Pickup» von D.R. Dill in der Zeitschrift TAPPI (Proceedings of the Technical Association of the Pulp and Paper Industry, Band 57, Nr. 1 vom Januar 1974, Seiten 97–100). Die Faserstoffsuspension, zu welcher die Leimungsmittel (A) und die Retentionsmittel (B) gegeben werden, weist in der Regel einen Feststoffgehalt von 0,1 bis 5, vorzugsweise 0,3 bis 3, insbesondere 0,3 bis 1 Gewichtsprozent und einen Schopper-Riegler-Mahlgrad von etwa 10° bis etwa 60°, vor allem 20 bis 60°, vorzugsweise 20 bis 45°, insbesondere 25 bis 35°, auf. Sie enthält in der Regel Zellstoff, insbesondere solchen aus Nadelholz, z.B. Kiefernholz, oder aus Hartholz, d.h. Laubholz, z.B. Buchenholz, der nach herkömmlichen Verfahren, z.B. dem Sulfit- oder vor allem dem Sulfatverfahren hergestellt wird. Zudem enthält die Faserstoffsuspension gegebenenfalls Holzschliff. Auch alaunhaltiges Altpapier kann in der Faserstoffsuspension enthalten sein. Auch Zellstoffsuspensionen, die nach dem sogenannten CMP- oder CTMP-Verfahren (Chemimechanical and chemithermomechanical pulping processes, vgl. z.B. Artikel «Developments in Refiner Mechanical Pulping» von S.A. Collicutt und Mitarbeitern in TAPPI, Band 64, Nr.6 vom Juni 1981, Seiten 57 bis 61) hergestellt werden, kommen in Betracht.

Die Faserstoffsuspension kann zudem organische oder mineralische Füllmittel enthalten. Als organische Füllmittel kommen u.a. synthetische Pigmente, z.B. Polykondensationsprodukte aus Harnstoff oder Melamin und Formaldehyd mit grossen spezifischen Oberflächen, die in hochdisperser Form vorliegen und z.B. in den britischen Patentschriften 1 043 937 und 1 318 244 beschrieben sind, als mineralische Füllmittel u.a. Montmorillonit, Titandioxid, Calciumsulfat und vor allem Talk, Kaolin und/oder Kreide (Calciumcarbonat) in Betracht. In der Regel enthält die Faserstoffsuspension 0 bis 40, vorzugsweise 5 bis 25, insbesondere 15 bis 20 Gewichtsprozent, bezogen auf den Feststoffgehalt der Faserstoffsuspension, an Trockensubstanz der Füllmittel der angegebenen Art.

Der pH-Wert der Faserstoffsuspension kann in einem weiten Bereich liegen, wobei z.B. Werte von 3,5 bis etwa 10 vorliegen können.

Bei Zusatz von z.B. Calciumcarbonat werden alkalische Faserstoffsuspensionen mit einem pH-Wert von etwa 7 bis etwa 9, vorzugsweise 7,5 bis 8,5, erhalten. Saure Faserstoffsuspensionen mit einem pH-Wert von 3,5 bis 7, vorzugsweise 5 bis 7, insbesondere 5 bis 6, können in Abwesenheit von Calciumcarbonat durch Zugabe von Säuren, z.B. Schwefel- oder Ameisensäure oder vor allem von z.B. latent sauren Sulfaten, wie z.B. Aluminiumsulfat (Alaun), erhalten werden.

Faserstoffsuspensionen, die kein Füllmittel enthalten, können in einem breiten pH-Bereich von z.B. 3,5 bis 10 vorliegen. Bevorzugt sind Faserstoffsuspensionen, die gegebenenfalls durch Zusatz von Kreide einen pH-Wert von etwa 7 bis etwa 9 aufweisen und insofern vorteilhaft sind, dass mögliche Korrosionserscheinungen an den empfindlichen Papiermaschinen ausgeschlossen werden. Zudem ist die Lagerfähigkeit von Papier oder Karton, das bei pH-Werten von 7 bis 9 der Faserstoffsuspension geleimt worden sind, gegenüber solchen, die bei pH-Werten von 3,5 bis 7 geleimt worden sind, deutlich überlegen.

Die Faserstoffsuspension kann auch Additive enthalten, wie z.B. Stärke oder ihre Abbauprodukte, welche die Faser/Faser- oder Faser/Füllmittel-Bindung erhöhen.

Auch hochmolekulare Polymere der Acrylsäurereihe, z.B. Polyacrylamide mit Molekulargewichten über 1 000 000, können zur Faserstoffsuspension als Hilfsmittel zum Zurückhalten feinster Zellstoff-Faserteilchen gegeben werden, wobei minimale Einsatzmengen von etwa 0,005 bis 0,02 Gewichtsprozent, bezogen auf Trockensubstanz des Polymers und den Feststoffgehalt der Faserstoffsuspensionen, genügend sind.

Die Faserstoffsuspension wird im erfindungsgemässen Massenleimungsverfahren auf an sich bekannte Weise auf Blattbildnern oder vorzugsweise kontinuierlich auf Papiermaschinen üblicher Bauart zu Papier oder Karton weiterverarbeitet. Nach einer Trocknung bei etwa 100 bis 140 °C während etwa 0,5 bis 10 Minuten werden Papiere eines variablen Flächengewichtes von z.B. 50 bis 200 $g/m^2$ erhalten.

Zur Oberflächenleimung des Papiers im erfindungsgemässen Verfahren wird die Leimflotte, welche die Komponenten (A) und (B) enthält, z.B. durch Aufsprühen, vorzugsweise durch Foulardieren, in der Regel bei Raumtemperatur (15–25 °C) auf das Papier aufgebracht. Anschliessend wird das imprägnierte Papier bei 60 bis 140 °C, vorzugsweise 90 bis 110 °C während 0,1 bis 10, vorzugsweise 2 bis 6 Minuten getrocknet. Nach dem Trocknen wird ein Papier erhalten, das einen Flächenauftrag an Leimungs- und Retentionsmittel von 50 bis 150, vorzugsweise 60 bis 120 $mg/m^2$ aufweist.

Bei dem erfindungsgemäss zu leimenden Papier handelt es sich um Papiere beliebiger Art mit beliebigen Flächengewichten, z.B. um Papier und Kartons aus gebleichter und ungebleichter Sulfit- oder Sulfat-Cellulose.

Wie eingangs erwähnt, enthält die wässrige Zusammensetzung zur Durchführung des erfindungsgemässen Papierleimungsverfahrens neben fakultativen üblichen Zusätzen das Leimungsmittel (A), sofern das Leimungsmittel und das Retentionsmittel (B) bei der Massenleimung separat zur Faserstoffsuspension gegeben werden. In diesem Fall enthält die Zubereitung das Leimungsmittel in der Regel ganz oder vor allem teilweise in Form seiner Salze (erhalten unter Mitverwendung von z.B. Ammoniak, eines Alkyl- oder Alkanolamins oder eines Alkalimetallhydroxids der angegebenen Art in den vorstehend angegebenen Verhältnissen). Im allgemeinen enthalten solche Zusammensetzungen 5 bis 30, vorzugsweise 5 bis 20 Gewichtsprozent an Trockensubstanz des mindestens teilweise in Salzform vorliegenden Leimungsmittels, bezogen auf das Gewicht der wässrigen Zusammensetzung.

Hingegen enthält die wässrige Zusammensetzung neben den fakultativen, üblichen Zusätzen, sofern das Leimungsmittel (A) und das Retentionsmittel (B) bei der Massenleimung gleichzeitig zur Faserstoffsuspension gegeben werden,

(A) 2 bis 40, vorzugsweise 5 bis 30, insbesondere 5 bis 10 Gewichtsprozent Leimungsmittel (berechnet als Trockensubstanz), bezogen auf das Gesamtgewicht der wässrigen Zusammensetzung, wobei das Leimungsmittel gegebenenfalls in Salzform vorliegt, und

(B) 0,1 bis 20, vorzugsweise 0,5 bis 10, insbesondere 3 bis 8 Gewichtsprozent Retentionsmittel (berechnet als Trockensubstanz), bezogen auf das Gesamtgewicht der wässrigen Zusammensetzung.

Die wässrigen Zusammensetzungen der angegebenen Art enthalten gegebenenfalls als übliche Zusätze oberflächenaktive Verbindungen, z.B. Dispergatoren oder ferner Emulgatoren und/oder wasserlösliche, organische Lösungsmittel. Als Dispergatoren und Emulgatoren kommen z.B. herkömmliche Ligninsulfonate, Lignincarboxylate, Carboxymethylcellulose, Ethylenoxydaddukte von Alkylphenolen, Fettaminen, Fettalkoholen oder Fettsäuren, Fettsäureester mehrwertiger Alkohole, substituierte Benzimidazole oder Kondensationsprodukte aus Formaldehyd und aromatischen Sulfonsäuren, vor allem Naphthalinsulfonsäuren, in Betracht. Weitere oberflächenaktive Verbindungen sind vorzugsweise die anionischen Tenside, insbesondere Sulfattenside, z.B. Diethanolaminlaurylsulfat, Natriumlaurylsulfat oder ethoxylierte Laurylsulfate. Mögliche wasserlösliche, organische Lösungsmittel sind aliphatische Ether mit 1 bis 10 Kohlenstoffatomen, z.B. Dioxan, Ethylenglykol-n-butylether oder Diethylenglykolmonobutylether oder Alkohole mit 1 bis 4 Kohlenstoffatomen, z.B. Isopropanol, Ethanol oder Methanol.

Sofern die wässrigen Zusammensetzungen Zusätze der angegebenen Art enthalten, beträgt das Mengenverhältnis (Komponente (A) ): (Zusätze) in der Zusammensetzung 1:0,02 bis 1:0,3 vorzugsweise 1:0,05 bis 1:0,1, bezogen auf Trockensubstanz der Leimungsmittel und der Zusätze.

Die Zusammensetzungen werden auf übliche Weise hergestellt, indem man das Leimungsmittel (A) zusammen mit dem Retentionsmittel (B) oder das Leimungsmittel (A) in der Regel teilweise in Form seines Salzes für sich allein entweder in geschmolzenem Zustand oder vorzugsweise in festem Zustand, insbesondere in pulverisierter Form, in der Regel in Gegenwart von Glasperlen und nötigenfalls von Emulgatoren (bei Leimungsmitteln in geschmolzenem Zustand) oder Dispergatoren (bei Leimungsmitteln in Pulverform) bei höchstens 90 °C, vorzugsweise etwa 50 bis 85 °C bei Emulsionen, insbesondere bei etwa 15 bis etwa 25 °C bei Dispersionen, verrührt, wobei lagerstabile, homogene, weiterverdünnbare Emulsionen oder vorzugsweise Dispersionen erhalten werden. Da die Leimungsmittel zusammen mit den Retentionsmitteln oder die ganz oder mindestens teilweise als Salze vorliegenden Leimungsmittel in der Regel selbst-dispergierend oder selbst-emulgierend sind, ist der Einsatz von Dispergatoren oder Emulgatoren im allgemeinen nicht unbedingt erforderlich. Dies gilt auch für den fakultativen Zusatz von Lösungsmitteln und/oder Tensiden, die nur bei ungenügender Lagerstabilität der Dispersionen oder Emulsionen eingesetzt werden.

Bei der Oberflächenleimung von Papier wird die dazu benötigte Leimflotte durch Verdünnen mit Wasser der vorstehend angegebenen Emulsionen oder Dispersionen, die sowohl das Leimungsmittel (A) als auch das Retensionsmittel (B) enthalten, hergestellt. Hierbei werden die Emulsionen oder Dispersionen so verdünnt, dass eine Leimflotte entsteht, die (A) 0,02 bis 0,4 vorzugsweise 0,05 bis 3, insbesondere 0,05 bis 1

Gewichtsprozent Leimungsmittel (berechnet als Trockensubstanz), bezogen auf das Gesamtgewicht der Leimflotte, wobei das Leimungsmittel gegebenenfalls in Salzform vorliegt, und (B) 0,01 bis 0,2, vorzugsweise 0,05 bis 0,1, insbesondere 0,3 bis 0,8 Gewichtsprozent Retentionsmittel (berechnet als Trockensubstanz), bezogen auf das Gesamtgewicht der wässrigen Leimflotte, enthält.

Als Vorteil des erfindungsgemässen Verfahrens sei erwähnt, dass in der Massenleimung Faserstoffsuspensionen der verschiedensten Art mit relativ besonders kleinen Mengen an Leimungs- und Retentionsmittel auf einfache Art und Weise zu Papier verarbeitet werden können, welches gute Leimungseigenschaften (Alkalitropfenprobe, Tintenschwimmdauer und vor allem Wasseraufnahme nach Cobb) aufweist. Dies gilt auch für die Oberflächenleimung, bei welcher die guten Leimungseffekte bereits mit geringen Flächenaufträgen an Leimungs- und Retentionsmittel erreicht werden. Insbesondere ermöglichen die geringen Flächenaufträge eine rasche Arbeitsweise, sodass bei der Trocknungstemperatur von z.B. 90 bis 110° bereits innerhalb etwa 20 bis 40 Sekunden gute Oberflächenleimungen erzielt werden. Das verfahrensgemäss in der Masse geleimte Papier weist gute mechanische Eigenschaften, d.h. gute Festigkeiten, insbesondere eine gute Reissfestigkeit auf. Eine gute Reproduzierbarkeit des Verfahrens sowohl bei der Massen- als auch bei der Oberflächenleimung ist gewährleistet. Insbesondere können bei der Massenleimung holzschliffhaltige oder altpapierhaltige Faserstoffsuspensionen verarbeitet werden. Auch die Kompatibilität des erfindungsgemäss verwendeten Leimungsmittels mit verschiedenen Füllmitteln wie z.B. Kaolin und auch verschiedenen Zusätzen, wie z.B. Alaun in saurem Bereich der Faserstoffsuspensionen in der Massenleimung, ist vorteilhaft. Die erfindungsgemäss verwendeten Leimungs- und Retentionsmittel weisen eine gute Kompatibilität mit den üblichen, in der Papierindustrie verwendeten Hilfsstoffen wie Farbstoffen, Pigmenten, Bindemitteln, insbesondere optischen Aufhellern und sonstigen Zusatzstoffen auf. Im weiteren neigen die eingesetzten Leimungs- und Retentionsmittel nicht zu einer unerwünschten Schaumbildung. Zudem wird der Weissgrad des geleimten Papiers durch die Leimung nicht wesentlich beeinflusst und kann sogar u.U. sowohl bei der Massen- als auch bei der Oberflächenleimung verbessert werden. Vor allem ist die in der Regel überraschend hohe Lagerstabilität der Leimungsmitteldispersionen der angegebenen Art von grossem Vorteil.

Die in den nachfolgenden Herstellungsvorschriften und Ausführungsbeispielen angegebenen Teile und Prozente beziehen sich auf das Gewicht.

Herstellungsvorschriften bekannter Verbindungen als Leimungsmittel

Vorschrift A:

Eine Suspension von 13,8 Teilen Salicylsäure (0,1 Mol) in 200 Teilen Toluol wird innerhalb von 15 Minuten mit einer Lösung von 30,2 Teilen Stearinsäurechlorid (0,1 Mol) in 100 Teilen Toluol versetzt. Das Reaktionsgemisch wird auf die Rückflusstemperatur von ca. 111 °C aufgeheizt und während 10 Stunden bei dieser Temperatur gehalten, wobei Chlorwasserstoffentwicklung zu beobachten ist. Danach wird das Lösungsmittel unter vermindertem Druck abdestilliert. Man erhält als weisses Pulver 38 Teile der Verbindung der Formel (11), die zu Analysenzwecken aus Aceton umkristallisiert werden kann. Smp.: 57–60 °C. Säurezahl: 142.

Vorschrift B:

Man verfährt wie in Vorschrift A angegeben, setzt jedoch 13,8 Teile 4-Hydroxybenzoesäure (0,1 Mol) (statt 13,8 Teilen Salicylsäure) ein. Man erhält als weisses Pulver 36 Teile der Verbindung der Formel (12), die zu Analysenzwecken aus Chloroform umkristallisiert werden kann. Smp.: 124–130 °C. Säurezahl: 135.

Vorschrift C:

Eine Suspension von 13,7 Teilen Anthranilsäure (0,1 Mol) in 150 Teilen Toluol wird mit einer Lösung von 30,2 Teilen Stearinsäurechlorid (0,1 Mol) in 50 Teilen Toluol, sodann mit einer Lösung von 20,2 Teilen Triethylamin (0,2 Mol) in 80 Teilen Toluol versetzt, wobei die Temperatur des Reaktionsgemisches von selber auf 45 °C steigt. Das Reaktionsgemisch wird auf die Rückflusstemperatur von ca. 111 °C aufgeheizt und während 1 Stunde bei dieser Temperatur gehalten. Beim Abkühlen des Reaktionsgemisches auf 20 °C fällt das Produkt als Salz aus, das abfiltriert und in 60 Teile Wasser eingetragen wird. Der pH-Wert der so erhaltenen, wässrigen Suspension wird mit einer 30%igen, wässrigen Salzsäurelösung auf 1,5 eingestellt. Der Ansatz wird danach noch während 4 Stunden unter Rühren gehalten wobei das Produkt als freie Säure ausfällt. Das Produkt wird abfiltriert und unter vermindertem Druck getrocknet. Man erhält als weisses Pulver 36 Teile der Verbindung der Formel (16), die zu Analysenzwecken aus Aceton umkristallisiert werden kann. Smp.: 105–107 °C. Säurezahl: 141.

Vorschrift D:

Man verfährt wie in Vorschrift C angegeben, setzt jedoch 13,7 Teile 3-Amino-benzoesäure (0,1 Mol) (statt 13,7 Teilen Anthranilsäure) ein. Man erhält als ockerfarbenes Pulver 34 Teile der Verbindung der Formel (17), die zu Analysenzwecken aus Dioxan umkristallisiert werden kann. Smp.: 191–194 °C. Säurezahl: 141.

Vorschrift E:

Eine Lösung von 10,9 Teilen 4-Aminophenol (0,1 Mol), 29,5 Teilen Octadecylisocyanat (0,1 Mol) und 0,2 Teilen 1,4-Diazabicyclo-(2,2,2)-octan (Katalysator) in 150 Teilen Toluol wird auf die Rückflusstemperatur von ca. 111 °C aufgeheizt und während 3 Stunden bei dieser Temperatur gehalten. Anschliessend wird das Lösungsmittel unter vermindertem Druck abdestilliert. Man erhält als hellgraues Pulver 38 Teile der Verbindung der Formel (31), die zu Analysenzwecken aus Dioxan umkristallisiert werden kann. Smp.: 141–143 °C. Säurezahl: 135.

Vorschrift F:

Eine Lösung von 43,8 Teilen Anilin-3-sulfonsäure (0,25 Mol) in 75,0 Teilen Wasser, 17,5 Teilen einer wässrigen 25%igen Ammoniaklösung und 91,0 Teilen Isopropanol wird mit 75,7 Teilen Stearinsäurechlorid (0,25 Mol) versetzt. Gleichzeitig werden aus einem separaten Zulaufgefäss 17,0 Teile einer wässrigen 25%igen Ammoniaklösung so zudosiert, dass der pH-Wert des Reaktionsgemisches zwischen 5 und 6 bleibt. Hierbei entsteht eine weisse Suspension, die eingedampft und getrocknet wird. Man erhält als braunes, wachsartiges Produkt 114 Teile der Verbindung der Formel (18). Smp.: 45–55 °C (Sintertemperatur).

Vorschrift G:

17,3 Teile Anilin-3-sulfonsäure (0,10 Mol) werden in 100 Teilen Pyridin bei 60 °C gelöst und mit 30,3 Teilen Stearinsäurechlorid (0,10 Mol) innerhalb von 20 Minuten versetzt, wobei sich die Temperatur des Reaktionsgemisches von selber auf 70° erhöht. Das Reaktionsgemisch wird auf 80 °C weiter aufgeheizt und während 6 Stunden bei dieser Temperatur unter Rühren gehalten. Zur erhaltenen, braunen Lösung werden 110 Teile eines wässrigen, 30%igen Salzsäurelösung zugegeben. Anschliessend wird das Reaktionsgemisch auf 90 °C weiter aufgeheizt und mit 400 Teilen entionisiertem Wasser versetzt, wobei das Produkt als brauner Niederschlag ausfällt. Das Produkt wird abfiltriert, mit 100 Teilen entionisiertem Wasser nachgewaschen und bei 50 °C unter vermindertem Druck getrocknet. Man erhält als braunes, wachsartiges Produkt 43, 7 Teile der Verbindung der Formel (19). Smp.: 40–50 °C (Sintertemperatur). Säurezahl: 166.

Vorschrift H:

Man verfährt wie in Vorschrift F angegeben, setzt jedoch 43,8 Teile Anilin-4-sulfonsäure (0,25 Mol) (statt 43,8 Teilen Anilin-3-sulfonsäure) ein. Man erhält als braunes, wachsartiges Produkt 97 Teile der Verbindung der Formel (20). Smp.: 50–60 °C (Sintertemperatur).

Vorschrift I, 1. Stufe:

23,6 Teile Anilin (0,25 Mol) werden bei 40 °C in 200 Teilen Pyridin gelöst. Innerhalb von 30 Minuten werden 75,5 Teile Stearinsäurechlorid (0,25 Mol) zugesetzt, wobei sich die Temperatur des Reaktionsgemisches von selber auf 55 °C erhöht. Anschliessend wird das Reaktionsgemisch auf 100 °C aufgeheizt und während 4 Stunden bei dieser Temperatur unter Rühren gehalten. Diese Lösung wird dann in 4000 Teile entionisiertes Wasser unter intensivem Rühren eingetragen, wobei das Produkt ausfällt. Die entstandene, hellbraune Suspension wird abfiltriert, das Produkt mit 1000 Teilen Wasser nachgewaschen und unter vermindertem Druck bei 40 °C getrocknet. Man erhält als braunstichiges, mehliges Pulver 88 Teile der Verbindung der Formel

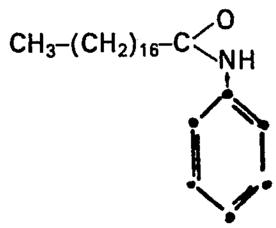

(56)

Smp.: 88–89 °C.

Vorschrift I, 2. Stufe:

36,0 Teile des gemäss Vorschrift I, 1. Stufe, als Zwischenprodukt erhaltenen Stearinsäureanilids (0,1 Mol) werden in 200 Teilen Chloroform gelöst und auf 30 °C aufgeheizt. Zu dieser Lösung werden in inerter Stickstoffatmosphäre 6,65 Teile Chlorsulfonsäure (0,1 Mol) innerhalb von 10 Minuten gegeben, wobei sich die Temperatur des Reaktionsgemisches von selber auf 45 °C erhöht und die durch die Reaktion freigesetzte Salzsäure aus dem Reaktionsgemisch entfernt wird. Das Reaktionsgemisch wird dann bei 30 °C während 1 Stunde unter Rühren gehalten, dann auf die Rückflusstemperatur von ca. 63 °C aufgeheizt und während 3 Stunden bei dieser Temperatur unter Rühren gehalten. Anschliessend wird das Lösungsmittel bei 40 °C unter vermindertem Druck abdestilliert und der Rückstand getrocknet. Man erhält als hellbraunes Wachs 50 Teile des Isomerengemisches der Formel (21). Smp.: 50–60 °C (Sintertemperatur). Säurezahl 138.

Vorschrift J:

54,6 Teile p-Aminophenol (0,5 Mol) werden in 200 Teilen Pyridin auf 50 °C aufgeheizt. Zu dieser Lösung werden innerhalb von 1 Stunde 151 Teile Stearinsäurechlorid (0,5 Mol) gegeben, wobei das Stearinsäurechlorid als 40 °C warme Schmelze eingesetzt wird. Die Temperatur des Reaktionsgemisches erhöht sich von selber auf 65 °C, wobei eine Suspension entsteht, die während 4 Stunden bei 50 °C unter Rühren gehalten wird. Das Reaktionsgemisch wird dann auf 90 °C weiter aufgeheizt und zu 4000 Teilen entionisiertem Wasser unter intensivem Rühren zugegeben. Die dunkelbraun gefärbte Suspension wird abfiltriert und das Produkt mit 300 Teilen Wasser nachgewaschen und bei 40 °C unter vermindertem Druck getrocknet. Man erhält als hell-violettes Pulver 147 Teile der Verbindung der Formel (22). Smp.: 120–122 °C. Säurezahl: 144.

Herstellungsbeispiele neuer Verbindungen als Leimungsmittel

Beispiel 1

Man verfährt wie in Vorschrift A angegeben, setzt jedoch 13,8 Teile 3-Hydroxy-benzoesäure (0,1 Mol) (statt 13,8 Teilen Salicylsäure) ein. Man erhält als weisses Produkt 36,3 Teile der Verbindung der Formel (13), die zu Analysenzwekken aus Aceton umkristallisiert werden kann. Smp.: 87–91 °C. Säurezahl: 139.

Beispiel 2

Eine Suspension von 13,7 Teilen 3-Amino-

benzoesäure (0,1 Mol) in 400 Teilen Toluol wird bei Raumtemperatur mit 29,5 Teilen Octadecylisocyanat (0,1 Mol) versetzt und auf die Rückflusstemperatur von ca. 111 °C aufgeheizt und während 1 Stunde bei dieser Temperatur gehalten. Danach wird das Lösungsmittel unter vermindertem Druck abdestilliert und der Rückstand getrocknet. Man erhält als ockerfarbenes Pulver 41 Teile der Verbindung der Formel (32), die zu Analysenzwecken aus Dioxan umkristallisiert werden kann. Smp.: 266 °C unter Zersetzung. Säurezahl: 132.

Beispiel 3

Eine Suspension von 13,8 Teilen 3-Hydroxybenzoesäure (0,1 Mol) und 0,2 Teilen 1,4-Diazabicyclo(2,2,2)-octan (Katalysator) in 200 Teilen Toluol wird bei 20 °C innerhalb von 10 Minuten mit 29,5 Teilen Octadecylisocyanat (0,1 Mol) versetzt. Das Reaktionsgemisch wird auf die Rückflusstemperatur von ca. 111 °C aufgeheizt und während 5 Stunden bei dieser Temperatur gehalten, wobei sich nach etwa 4 Stunden eine klare Lösung bildet. Anschliessend wird das Lösungsmittel unter vermindertem Druck abdestilliert und der Rückstand unter vermindertem Druck getrocknet. Man erhält als weisses Pulver 40,8 Teile der Verbindung der Formel (26), die zu Analysenzwecken aus einem Gemisch von Aceton und Hexan umkristallisiert werden kann. Smp.: 112–122 °C. Säurezahl 116.

Beispiel 4

Man verfährt wie in Beispiel 3 angegeben, setzt jedoch 13,8 Teile 4-Hydroxy-benzoesäure (0,1 Mol) (statt 13,8 Teile 3-Hydroxy-benzoesäure) ein. Man erhält als weisses Pulver 42,3 Teile der Verbindung der Formel (33), die zu Analysenzwecken aus Dioxan umkristallisiert werden kann. Smp.: 177–183 °C. Säurezahl: 119.

Beispiel 5

27,5 Teile Resorcin (0,25 Mol) und 8,7 Teile Pyridin (0,11 Mol) werden bei 50 °C in 100 Teilen Tetrahydrofuran gelöst. Dieser Lösung wird bei 50 °C innerhalb von 30 Minuten eine Lösung von 30,3 Teilen Stearinsäureachlorid (0,1 Mol) in 50 Teilen Tetrahydrofuran zugegeben. Das Reaktionsgemisch wird auf die Rückflusstemperatur von ca. 67 °C aufgeheizt, während 4 Stunden bei dieser Temperatur unter Rühren gehalten und dann auf 25 °C abgekühlt. Zum Reaktionsgemisch werden 250 Teile einer wässrigen, 0,5 N Salzsäurelösung gegeben, wobei das Produkt ausfällt. Das Produkt wird abfiltriert, mit 500 Teilen Wasser von 60 °C nachgewaschen und unter vermindertem Druck bei 60 °C getrocknet. Man erhält als weisses Pulver 33,5 Teile der Verbindung der Formel (14). Smp.: 52–57 °C. Säurezahl: 147.

Beispiel 6

58,6 Teile Stearinsäure (0,2 Mol) und 22,0 Teile Resorcin (0,2 Mol) werden auf 120 °C aufgeheizt. Die entstandene, klare Schmelze wird mit 0,2 Teilen einer 96%igen Schwefelsäure versetzt und auf 160 °C aufgeheizt. Das durch die Reaktion gebildete Wasser wird im Stickstoffstrom ausgetrieben. Das Reaktionsgemisch wird insgesamt während 2 Stunden bei 160 °C unter Rühren gehalten und dann auf 20 °C abgekühlt. Man erhalt als orange-farbenes Pulver 74 Teile eines Rohproduktes, das die Verbindung der in Beispiel 5 angegebenen Formel (14) enthält. Smp.: 73–82 °C. Säurezahl: 143.

Beispiel 7

Man verfährt wie in Beispiel 5 angegeben, setzt jedoch 27,5 Teile Brenzkatechin (0,25 Mol) (statt 27,5 Teile Resorcin) ein. Man erhält als weisses Pulver 36,4 Teile der Verbindung der Formel (15). Smp.: 62–66 °C. Säurezahl: 152.

Beispiel 8

22,0 Teile Brenzkatechin (0,2 Mol) und 1,1 Teile Triethylamin werden in 100 Teilen Tetrahydrofuran gelöst und auf 60 °C aufgeheizt. Zu dieser Lösung werden bei dieser Temperatur 59,1 Teile Stearylisocyanat (0,2 Mol) innerhalb von 20 Minuten gegeben. Das Reaktionsgemisch wird anschliessend während 6 Stunden bei 60 °C unter Rühren gehalten. Das Lösungsmittel wird dann unter vermindertem Druck bei 40 °C abdestilliert und der Rückstand getrocknet. Man erhält als dunkelbraunes Pulver 79,0 Teile der Verbindung der Formel (27). Smp.: 73–78 °C. Säurezahl: 142.

Beispiel 9

Man verfährt wie in Beispiel 8 angegeben, setzt jedoch 22,0 Teile Resorcin (0,2 Mol) (statt 22,0 Teilen Brenzkatechin) ein. Man erhält als hellbraunes Pulver 78,2 Teile der Verbindung der Formel (28). Smp.: 83–86°. Säurezahl: 140.

Beispiel 10

Zu einer Lösung von 90,5 Teilen Octadecylisocyanat (0,3 Mol) in 200 Teilen Tetrahydrofuran wird bei 50 °C eine Lösung von 32,8 Teilen 2-Aminophenol (0,3 Mol) in 250 Teilen Tetrahydrofuran innerhalb von 30 Minuten gegeben. Das Reaktionsgemisch wird anschliessend während 16 Stunden bei 50 °C unter Rühren gehalten, wobei eine hellbraun gefärbte Suspension entsteht. Die Suspension wird auf 5 °C abgekühlt und abfiltriert. Das erhaltene Produkt wird bei 40° unter vermindertem Druck getrocknet. Man erhält als beiges Pulver 30 Teile der Verbindung der Formel (34), die zu Analysenzwecken aus Ethanol umkristallisiert werden kann. Smp.: 90–93 °C. Säurezahl: 139.

Beispiel 11

Man verfährt wie in Beispiel 10 angegeben, setzt jedoch 32,8 Teile 3-Aminophenol (0,3 Mol) (statt 32,8 Teile 2-Aminophenol) ein. Man erhält als gelbliches Pulver 96 Teile der Verbindung der Formel (35), die zu Analysenzwecken aus Ethanol umkristallisiert werden kann. Smp.: 108–111 °C. Säurezahl: 138.

Beispiel 12

Zu einer Lösung von 17,3 Teilen Anilin-2-sulfonsäure (0,1 Mol) in 100 Teilen Pyridin werden 29,6 Teile Stearylisocyanat (0,1 Mol) innerhalb von 20 Minuten bei 70 °C gegeben. Die erhaltene, klare Lösung wird auf 80 °C aufgeheizt und während 5 Stunden bei dieser Temperatur unter Rühren gehalten. Nach der Zugabe von 110 Teilen einer 30%igen, wässrigen Salzsäurelösung wird das Reaktionsgemisch auf 100 °C weiter aufgeheizt. Nun wird das Reaktionsgemisch mit 400 Teilen entionisiertem Wasser versetzt, wobei eine weisse Suspension entsteht. Diese Suspension wird auf 20 °C abgekühlt und abfiltriert. Das Produkt wird mit 300 Teilen entionisiertem Wasser nachgewaschen und bei 40 °C unter vermindertem Druck getrocknet. Man erhält als hellgrau gefärbtes Pulver 35,2 Teile der Verbindung der Formel (36). Smp.: 71–73 °C. Säurezahl: 129.

Beispiel 13

Man verfährt wie in Beispiel 12 angegeben, setzt jedoch 17,3 Teile Anilin-4-sulfonsäure (0,1 Mol) (statt 17,3 Teilen Anilin-2-sulfonsäure) ein. Man erhält als rosagefärbtes, wachsartiges Produkt 43,5 Teile der Verbindung der Formel (37), Smp.: ca. 65 °C (Sintertemperatur). Säurezahl: 122.

Beispiel 14

17,5 Teile 4-Aminophenol (0,16 Mol) werden in 400 Teilen einer 96%igen Schwefelsäure bei 0 °C gelöst und bei dieser Temperatur portionsweise innerhalb von 4 Stunden mit 50,2 Teilen N-Hydroxymethyl-stearinsäureamid (0,16 Mol) versetzt. Das Reaktionsgemisch wird während 48 Stunden bei 20 °C unter Rühren gehalten, dann auf Eis gegossen, worauf eine Suspension entsteht, welche abfiltriert wird. Das Produkt wird bei 40 °C unter vermindertem Druck getrocknet. Man erhält als graues Pulver 41 Teile der Verbindung der Formel (9), die zu Analysenzwecken aus Ethanol umkristallisiert wird. Smp.: 150–160 °C. Säurezahl: 145.

Beispiel 15

Man verfährt wie in Beispiel 14 angegeben, setzt jedoch 22,6 Teile 4-Nitrophenol (0,16 Mol) (statt 17,5 Teilen 4-Aminophenol) ein. Man erhält als graues Pulver 40 Teile der Verbindung der Formel (10), die zu Analysenzwecken aus einem Gemisch von Ethanol und Aceton umkristallisiert werden kann. Smp.: 112–114 °C.Säurezahl: 136.

Beispiel 16

Eine Lösung von 44,0 Teilen Hydrochinon (0,4 Mol), 29,5 Teilen Octadecylisocyanat (0,1 Mol) und 0,2 Teilen 1,4-Diazabicyclo-(2,2,2)-octan (Katalysator) in 200 Teilen Toluol wird auf die Rückflusstemperatur von ca. 111 °C aufgeheizt und während 6 Stunden bei dieser Temperatur unter Rühren gehalten. Das Reaktionsgemisch wird dann auf 20 °C abgekühlt, wobei das Produkt ausfällt. Das Produkt wird abfiltriert, mit 300 Teilen Wasser von 60 °C nachgewaschen und unter vermindertem Druck getrocknet. Man erhält als weisses Pulver 35,5 Teile der Verbindung der Formel (29). Smp.: 84–87 °C. Säurezahl: 135.

Beispiel 17

Eine Lösung von 17,7 Teilen Pyrogallol (0,14 Mol) in 100 Teilen Toluol und 150 Teilen Dimethylsulfoxid wird bei 20 °C mit 14,5 Teilen Triethylamin (0,14 Mol) und dann mit einer Lösung von 41,4 Teilen Octadecylisocyanat (0,14 Mol) in 50 Teilen Toluol versetzt. Das Reaktionsgemisch wird auf 45 °C aufgeheizt, während 2 Stunden bei dieser Temperatur unter Rühren gehalten und anschliessend zu 500 Teilen einer wässrigen, 2 N Salzsäurelösung gegeben, wobei das Produkt ausfällt. Das Produkt wird abfiltriert, mit Wasser gewaschen und unter vermindertem Druck getrocknet. Man erhält als leicht gelbliches Pulver 37 Teile der Verbindung der Formel (30), die zu Analysenzwecken aus Ethanol umkristallisiert werden kann. Smp.: 83–85 °C. Säurezahl: 261.

Beispiel 18

Eine Suspension von 28,8 Teilen 1,5-Dihydroxynaphthalin (0,18 Mol) in 200 Teilen Toluol wird bei 20 °C innerhalb von 20 Minuten mit einer Lösung von 53,2 Teilen Octadecylisocyanat (0,18 Mol) in 50 Teilen Toluol und sodann mit 18,2 Teilen Triethylamin (0,18 Mol) versetzt. Das Reaktionsgemisch wird auf die Rückflusstemperatur von ca. 111 °C aufgeheizt und während 16 Stunden bei dieser Temperatur unter Rühren gehalten, wobei eine klare Lösung entsteht. Diese Reaktionslösung wird dann auf 20 °C abgekühlt, wobei das Produkt ausfällt. Das Produkt wird abfiltriert, mit einer wässrigen, 1 N Salzsäurelösung und dann mit Wasser nachgewaschen und schliesslich unter vermindertem Druck getrocknet. Man erhält als hellbraunes Pulver 63 Teile der Verbindung der Formel (38), die zu Analysenzwecken aus Chlorbenzol umkristallisiert werden kann. Smp.: 153–156 °C. Säurezahl: 122.

Beispiel 19

Eine Suspension von 32 Teilen 1,5-Dihydroxynaphthalin (0,2 Mol) in 200 Teilen Toluol wird bei Raumtemperatur zuerst mit 16,1 Teilen Pyridin (0,2 Mol), dann innerhalb von 40 Minuten mit einer Lösung von 60,2 Teilen Ölsäurechlorid (0,2 Mol) in 50 Teilen Toluol versetzt. Das Reaktionsgemisch wird auf die Rückflusstemperatur von ca. 111 °C aufgeheizt und während 16 Stunden bei dieser Temperatur unter Rühren gehalten. Anschliessend wird das Lösungsmittel unter vermindertem Druck abdestilliert. Der Destillationsrückstand wird in 250 Teilen Chloroform gelöst und mit 100 Teilen einer wässrigen, 1 N Salzsäurelösung und dann mit 100 Teilen Wasser nachgewaschen. Die Chloroformlösung wird mit Magnesiumsulfat getrocknet und unter vermindertem Druck zur Trockene eingedampft. Man erhält als zähflüssiges Produkt 82 Teile der Verbindung der Formel (25). Säurezahl: 129.

Beispiel 20

Man verfährt wie in Vorschrift J angegeben, setzt jedoch 54,6 Teile o-Aminophenol (0,5 Mol) (statt 54,6 Teilen p-Aminophenol) ein. Man erhält als hellbeiges Pulver 182 Teile der Verbindung der Formel (23). Smp.: 75–77 °C. Säurezahl: 142.

Beispiel 21

Man verfährt wie in Vorschrift J angegeben, setzt jedoch 54,6 Teile m-Aminophenol (0,5 Mol) (statt 54,6 Teilen p-Aminophenol) ein. Man erhält als hellbeiges Pulver 178 Teile der Verbindung der Formel (24). Smp.: 102–104 °C. Säurezahl: 140.

Beispiel 22

22,0 Teile Brenzkatechin (0,2 Mol) werden bei 110 °C geschmolzen. Zu dieser Schmelze werden 60,5 Teile Stearinsäurechlorid (0,2 Mol) innerhalb von ca. 30 Minuten so gegeben, dass die Temperatur nicht über 120 °C steigt. Die lebhaft entweichende Salzsäure wird durch Einleiten in eine wässrige verdünnte Natriumhydroxidlösung vernichtet. Das Reaktionsgemisch wird auf 150 °C weiter aufgeheizt und bei dieser Temperatur während ca. 30 Minuten unter Rühren gehalten, bis keine Salzsäure mehr entweicht. Die geschmolzene Reaktionsmasse wird auf Wasser ausgegossen. Nach dem Filtrieren und Zerkleinern erhält man als hellrosa gefärbtes Pulver 72,4 Teile der Verbindung der in Beispiel 7 angegebenen Formel (15). Smp.: 62–68 °C, Säurezahl 151.

Applikationsbeispiele

Beispiele 23 bis 48

Eine Faserstoffsuspension aus gebleichtem Birkensulfatzellstoff und Kiefernsulfatzellstoff im Gewichtsverhältnis 1:1 in Wasser von 10° dH (deutsche Härtegrade), die einen Schopper-Riegler-Mahlgrad von 35° und einen Feststoffgehalt von 0,5% aufweist, wird mit 20% Kreide als Füllmittel und hierauf mit 0,01% PERCOL 292® (kationisches, hochmolekulares (MG $>1\cdot10^7$) Polyacrylamid) als Hilfsmittel zum Zurückhalten feinster Zellstoffaserteilchen versetzt, wobei sich der in der nachfolgenden Tabelle I angegebene pH-Wert der Faserstoffsuspension einstellt. Die Prozentangaben beziehen sich auf Trockensubstanz an Hilfs- und Füllmittel, bezogen auf den Feststoffgehalt der Faserstoffsuspension.

Formulierungen des Leimungsmittels werden hergestellt, indem jeweils 7% der angegebenen Leimungsmittel in Pulverform, wie sie bei der Herstellung anfallen, mit jeweils 3,5% POLYMIN® P (Polyethylenimin eines Molekulargewichts von 10 000 bis 100 000) als Retentionsmittel in Gegenwart von entionisiertem Wasser und von Glasperlen mit einem Durchmesser von 2 mm bei Raumtemperatur (15 bis 25 °C) verrührt werden. Die erhaltenen Dispersionen sind giessbar, homogen und lagerstabil. Die Prozentangaben beziehen sich auf die Trockensubstanz an Leimungs- und Retentionsmittel, bezogen auf das Gesamtgewicht der Formulierung.

Nun wird die wässrige Formulierung des Leimungsmittels und des Retentionsmittels zur Faserstoffsuspension so gegeben, dass 0,5% an Trockensubstanz des Leimungsmittels, bezogen auf den Feststoffgehalt der Faserstoffsuspension, entsteht. Anschliessend wird die Faserstoffsuspension in einem Labor-Blattbildner «Formette Dynamique» der Fa. Allimand, Grenoble, Frankreich, zu Papierblättern verarbeitet, die nach der Trocknung bei 130 °C während 3 Minuten ein Flächengewicht von 80 g/m² aufweisen.

Beide Oberflächen der erhaltenen Papierblätter, d.h. die auf der Siebseite des Blattbildners erhaltene Oberfläche und die Gegen- oder Oberseite werden auf ihre Leimungseigenschaften geprüft. Zu diesem Zweck wird die Wasseraufnahme nach Cobb bei 30 Sekunden Einwirkungsdauer (WA $Cobb_{30}$) gemäss DIN 53 132 gemessen. Die Ergebnisse der WA $Cobb_{30}$-Messungen in g/m² der Siebseite (SS) und Oberseite (OS) nach der Trocknung bei 130 °C und nach einer Lagerung von einem Tag bei 23 °C und 50% relativer Feuchtigkeit sind in der nachfolgenden Tabelle I angegeben. Je geringer die Wasseraufnahme, desto besser ist die Leimung des Papiers. WA $Cobb_{30}$ Werte über 100 entsprechen einer völlig unbefriedigenden Leimung des Papiers.

Tabelle I

| Beispiel Nr. | Leimungsmittel | pH-Wert der Faserstoff-suspension | WA $Cobb_{30}$ (g/m²) nach Trocknung SS | nach Trocknung OS | nach 1 Tag Lagerung SS | nach 1 Tag Lagerung OS |
|---|---|---|---|---|---|---|
| 23 | Verbindung gemäss Vorschrift A | 8,7 | 23 | 14 | 24 | 14 |
| 24 | Verbindung gemäss Vorschrift B | 8,7 | 17 | 13 | 17 | 14 |
| 25 | Verbindung gemäss Vorschrift D | 8,5 | 21 | 16 | 24 | 17 |
| 26 | Verbindung gemäss Vorschrift E | 8,2 | 56 | 20 | 21 | 12 |
| 27 | Verbindung gemäss Vorschrift G | 9,3 | 69 | 19 | 40 | 15 |

Tabelle I (Fortsetzung)

| Beispiel Nr. | Leimungsmittel | pH-Wert der Faserstoff-suspension | WA $Cobb_{30}$ (g/m²) nach Trocknung SS | nach Trocknung OS | nach 1 Tag Lagerung SS | nach 1 Tag Lagerung OS |
|---|---|---|---|---|---|---|
| 28 | Verbindung gemäss 2. Stufe der Vorschrift I | 8,1 | 19 | 14 | 18 | 11 |
| 29 | Verbindung gemäss Vorschrift J | 8,7 | 52 | 14 | 20 | 12 |
| 30 | Verbindung gemäss Beispiel 1 | 8,7 | 17 | 13 | 18 | 14 |
| 31 | Verbindung gemäss Beispiel 2 | 8,7 | 47 | 18 | 43 | 15 |
| 32 | Verbindung gemäss Beispiel 3 | 7,9 | 20 | 15 | 19 | 16 |
| 33 | Verbindung gemäss Beispiel 4 | 7,9 | 17 | 14 | 16 | 13 |
| 34 | Verbindung gemäss Beispiel 7 | 7,9 | 16 | 14 | 16 | 12 |
| 35 | Verbindung gemäss Beispiel 8 | 9,2 | 32 | 15 | 27 | 12 |
| 36 | Verbindung gemäss Beispiel 9 | 9,2 | 35 | 15 | 30 | 12 |
| 37 | Verbindung gemäss Beispiel 10* | 8,2 | 31 | 16 | 18 | 12 |
| 38 | Verbindung gemäss Beispiel 11 | 8,4 | 66 | 16 | 42 | 12 |
| 39 | Verbindung gemäss Beispiel 13 | 9,4 | 25 | 13 | 19 | 13 |
| 40 | Verbindung gemäss Beispiel 14 | 8,7 | 44 | 14 | 26 | 12 |
| 41 | Verbindung gemäss Beispiel 15 | 8,7 | 24 | 14 | 25 | 13 |
| 42 | Verbindung gemäss Beispiel 16 | 8,2 | 26 | 15 | 26 | 14 |
| 43 | Verbindung gemäss Beispiel 17 | 9,0 | 20 | 14 | 19 | 12 |
| 44 | Verbindung gemäss Beispiel 18 | 9,1 | 26 | 15 | 22 | 13 |
| 45 | Verbindung gemäss Beispiel 19** | 9,1 | 38 | 18 | 18 | 13 |
| 46 | Verbindung gemäss Beispiel 20 | 8,7 | 22 | 11 | 15 | 11 |
| 47 | Verbindung gemäss Beispiel 21 | 8,8 | 32 | 13 | 32 | 12 |
| 48 | Verbindung gemäss Beispiel 22*** | 8,3 | 21 | 12 | 20 | 9 |

*Formulierung in Gegenwart von 0,35% Natriumlaurylsulfat (zusätzlich zu 7% Leimungsmittel und 3,5% POLYMIN® P)

** Formulierung als Schmelzemulsion in Gegenwart von 0,35% Natriumlaurylsulfat (zusätzlich zu 7% Leimungsmittel in geschmolzenem Zustand und 3,5% POLYMIN® P)

*** Formulierung mit einer wässrigen Essigsäurelösung auf den pH-Wert von 7,0 eingestellt

Ähnliche Ergebnisse werden erzielt, wenn man anstelle von POLYMIN® P als Retentionsmittel CATO® 110 (kationisch modifizierte Stärke, die mit einem quaternären Ammoniumgruppen enthaltenden Propylenoxid modifiziert ist und deren pH-Wert der 25%igen Anschlämmung in destilliertem Wasser bei 20 °C 4,2 bis 4,6 beträgt), POSAMYL® E7 (kationisch modifizierte Stärke mit einem Stickstoffgehalt von 0,4%), HOFFMANN® B 118 (mit Trimethylglycidylammoniumchlorid kationisch modifizierte, native Kartoffelstärke, deren Stickstoffgehalt 1,3% beträgt, ein Kondensationsprodukt aus Dicyandiamid und Triethylentetramin, das mit Epichlorhydrin weiter umgesetzt und gemäss Beispiel 2 der deutschen Offenlegungsschrift 2 710 061 hergestellt wird, ein Epichlorhydrinaddukt eines Umsetzungsproduktes aus Diethylendiamin und Adipinsäure, das gemäss Beispiel 1 der britischen Patentschrift 865 727 hergestellt wird, ein Umsetzungsprodukt aus Dicyandiamid, Formaldehyd, Ammoniumchlorid und Ethylendiamin, das gemäss Beispiel 1 der US-Patentschrift 3 491 064 hergestellt wird, oder RETAMINOL® K (Polyethylenimin eines Molekulargewichtes von 20 000 bis 40 000) einsetzt. Auch Gemische der Retentionsmittel der vorstehend angegebenen Art kommen hierbei in Betracht. Zur Erzielung guter Ergebnisse ist nötigenfalls ein Zusatz von Dispergatoren, insbesondere von Kondensationsprodukten aus Formaldehyd und Naphthalinsulfonsäuren oder von Carboxymethylcellulose vorteilhaft. Hingegen wird nur eine schlechte Leimung mit Cobb-Werten von etwa 150 bis etwa 250 erhalten, wenn man ein Leimungsmittel gemäss Vorschrift A, B, D, E, G, I, J oder gemäss einem der Beispiele 1 bis 4, 7 bis 11 oder 13 bis 22, jedoch kein Retentionsmittel, oder ein Retentionsmittel der vorstehend angegebenen Art, jedoch kein Leimungsmittel einsetzt.

Beispiele 49 bis 60

Man verfährt wie in Beispielen 23 bis 48 angegeben, gibt jedoch das Leimungsmittel und das Retentionsmittel separat zur Faserstoffsuspension, wobei 6, 7, 10 oder 15% Leimungsmittel in Pulverform bei Raumtemperatur (15 bis 25 °C) in Gegenwart von Wasser und Glasperlen mit einer wässrigen, 5%igen Ammoniaklösung zu einer selbstemulgierenden, weiterverdünnbaren, ebenfalls giessbaren, homogenen und lagerstabilen Emulsion verrührt werden und wobei die in der nachfolgenden Tabelle II angegebenen Formulierungen des Leimungsmittels entstehen. Gegebenenfalls können auch salzhaltige, ebenfalls selbstemulgierende, giessbare und lagerstabile Emulsionen des Leimungsmittels, wie sie bei der Herstellung anfallen, ohne Ammoniakzusatz direkt eingesetzt werden. Die angegebenen Val% bedeuten die Anzahl Aquivalente an Ammoniak für 100 Äquivalente, bezogen auf die Anzahl vorhandener, acider Gruppen der jeweils eingesetzten Leimungsmittel. 10 Sekunden nach der Zugabe der angegebenen Einsatzmenge an Trockensubstanz POLYMIN® P als Retentionsmittel wird die Faserstoffsuspension mit jeweils der angegebenen Einsatzmenge an Trockensubstanz des Leimungsmittels versetzt, wobei sich die Einsatzmenge an Leimungs- und Retentionsmittel auf den Feststoffgehalt der Faserstoffsuspension beziehen. Die Leimungsergebnisse sind ebenfalls aus der Tabelle II zu entnehmen.

Tabelle II

| Beispiel Nr. | Leimungsmittel Formulierung | Einsatzmenge Leimungsmittel (%) | Einsatzmenge Retentionsmittel (%) | pH-Wert der Faserstoffsuspension | WA $Cobb_{30}$ ($g/m^2$) nach Trocknung SS | nach Trocknung OS | nach 1 Tage Lagerung OS | nach 1 Tage Lagerung SS |
|---|---|---|---|---|---|---|---|---|
| 49 | 15% Verbindung gemäss Vorschrift B 60 Val% Ammoniak | 0,30 | 0,25 | 8,8 | 18 | 15 | 19 | 14 |
| 50 | 15% Verbindung gemäss Vorschrift C 100 Val% Ammoniak | 0,50 | 0,25 | 8,1 | 33 | 23 | – | – |
| 51 | 15% Verbindung gemäss Vorschrift D 100 Val% Ammoniak | 0,50 | 0,25 | 8,5 | 21 | 16 | 24 | 17 |
| 52 | 10% Verbindung gemäss Vorschrift F * | 0,50 | 0,25 | 8,5 | 21 | 18 | 25 | 19 |
| 53 | 10% Verbindung gemäss Vorschrift H | 0,50 | 0,25 | 8,6 | 36 | 36 | – | – |
| 54 | 15% Verbindung gemäss Beispiel 1 60 Val% Ammoniak | 0,30 | 0,25 | 8,8 | 18 | 15 | 19 | 14 |
| 55 | 10% Verbindung gemäss Beispiel 4 100 Val% Ammoniak | 0,15 | 0,15 | 8,4 | 27 | 20 | 22 | 19 |
| 56 | 10% Verbindung gemäss Beispiel 5 100 Val% Ammoniak | 0,15 | 0,15 | 8,6 | 27 | 24 | 23 | 20 |
| 57 | 10% Verbindung gemäss Beispiel 6 | 0,15 | 0,15 | 8,3 | 30 | 25 | 28 | 23 |

Tabelle II (Fortsetzung)

| Beispiel Nr. | Leimungsmittel Formulierung | Einsatzmenge Leimungsmittel (%) | Einsatzmenge Retentionsmittel (%) | pH-Wert der Faserstoffsuspension | Wa $Cobb_{30}$ ($g/m^2$) nach Trocknung SS | nach Trocknung OS | nach 1 Tag Lagerung OS | nach 1 Tag Lagerung SS |
|---|---|---|---|---|---|---|---|---|
| | 100% Val% Ammoniak | | | | | | | |
| 58 | 10% Verbindung gemäss Beispiel 7** 100 Val% Ammoniak | 0,15 | 0,15 | 8,5 | 25 | 23 | 22 | 20 |
| 59 | 7% Verbindung gemäss Beispiel 12 100 Val% Ammoniak | 0,50 | 0,25 | 9,3 | 29 | 29 | 30 | 26 |
| 60 | 6% Verbindung gemäss Beispiel 22** 100 Val% Ammoniak | 0,50 | 0,25 | 8,2 | 17 | 12 | 15 | 10 |

* Als ammoniumchloridhaltige, 10%ige Suspension ohne Ammoniakzusatz
** In Gegenwart von 0,5% Lignincarboxylat als Dispergierhilfsmittel

Bei Verwendung von 10 bis 200 Val% Ammoniak oder Natriumhydroxid (als 5%ige wässrige Lösung) zur Formulierung des Leimungsmittels werden ähnlich gute Leimungsergebnisse wie die in Tabelle II angegebenen erhalten.

Ähnliche Ergebnisse werden auch erzielt, wenn man zur Faserstoffsuspension das Leimungsmittel zuerst und 10 Sekunden hierauf das Retentionsmittel gibt. Das gleiche gilt auch, wenn auf die Zugabe von PERCOL® 292 und/oder auf die Zugabe eines Füllmittels verzichtet wird. Ähnliche Ergebnisse werden ebenfalls erzielt, wenn man anstelle von Kreide als Füllmittel Talk oder Kaolin oder wenn man zusätzlich Alaun einsetzt. Auch bei Einsatz von holzschliffhaltigen Faserstoffsuspensionen werden gute Leimungsergebnisse erhalten.

Beispiel 61

Ein Filterpapier aus reiner Cellulose mit einem Flächengewicht von 110 $g/m^2$ wird mit einer Geschwindigkeit von 3 m/Minute und eine Walzenpressung von 20 kp/cm mit einer wässrigen Flotte foulardiert, welche im Liter 18,4 g einer wässrigen Dispersion aus 7% der Verbindung gemäss Beispiel 22 als Leimungsmittel und 3,5% POLYMIN® P als Retentionsmittel, die mit einer wässrigen Essigsäurelösung auf den pH-Wert von 7,0 eingestellt worden ist, enthält. Hierbei wird die Dispersion des Leimungsmittels und des Retentionsmittels wie in Beispielen 23 bis 48 angegeben hergestellt.

Die Flottenaufnahme beträgt 78% (0,1% Einsatzmenge an reinem Leimungsmittel, bezogen auf das zu behandelnde Papier). Das foulardierte Papier wird zwischen zwei Filterpapierblättern während 3 Minuten bei 90 °C getrocknet. Das getrocknete, behandelte Papier weist einen WA $Cobb_{30}$ Wert von 19 $g/m^2$. Nach 1 Tag Lagerung beträgt der WA $Cobb_{30}$ Wert des behandelten Papiers 16 $g/m^2$. Ähnliche Ergebnisse werden auch erzielt, wenn man Dispersionen oder Schmelzemulsionen einsetzt, die als Leimungsmittel an Stelle der Verbindung gemäss Beispiel 22 eine Verbindung gemäss einer der Vorschriften H bis J oder gemäss einem der Beispiele 1 bis 21 enthalten.

**Patentansprüche**

1. Verfahren zum Leimen von Papier oder Karton, dadurch gekennzeichnet, dass man mindestens

(A) ein Leimungsmittel, das aus einer aromatischen Verbindung besteht, die am aromatischen Kern einen einzigen hydrophoben Substituenten mit mindestens 5 Kohlenstoffatomen und mindestens eine anionische, acide oder in Salzform vorliegende Gruppe aufweist, wobei der hydrophobe Substituent mit dem aromatischen Kern über ein Ester-, Amid-, Urethan- oder Harnstoffbrückenglied verknüpft, bei Ester- und Amidbrückengliedern die CO-Einheit an den hydrophoben Rest gebunden und zwischen den Brückengliedern und dem aromatischen Kern gegebenenfalls eine Methylengruppe vorhanden ist, und

(B) ein polymeres, kationisches Retentionsmittel einsetzt.

2. Verfahren nach Anspruch 1, dadurch gekennzeichnet, dass man zur Massenleimung von Papier oder Karton die Komponenten (A) und (B) zu wässrigen, cellulosehaltigen, gegebenenfalls füllmittelhaltigen Faserstoffsuspensionen in beliebiger Reihenfolge oder gleichzeitig gibt.

3. Verfahren nach Anspruch 1, dadurch gekennzeichnet, dass man zur Papieroberflächenleimung das Papier mit einer wässrigen Leimflotte, welche die Komponenten (A) und (B) enthält, imprägniert und trocknet.

4. Verfahren nach einem der Ansprüche 1 bis 3, dadurch gekennzeichnet, dass man als Komponente (A) ein Leimungsmittel der Formel

$$\begin{array}{l}(A_2)_{m-1}\\ \quad | \\ A_1-D_1-(CH_2)_{n-1}-X_1-R_1\end{array}$$

einsetzt, worin

$A_1$ und $A_2$ unabhängig voneinander jeweils eine anionische, in Salzform vorliegende oder acide Carboxyl-, Hydroxyl- oder Sulfogruppe,

$D_1$ durch Halogen, Nitro, Amino oder Hydroxyl substituiertes oder unsubstituiertes Phenylen, Naphthylen, Dihydronaphthylen oder Tetrahydronaphthylen,

$R_1$ Alkyl oder Alkenyl mit 5 bis 22 Kohlenstoffatomen,

$X_1$ ein Brückenglied der Formel –O–CO–, –NH–CO–, –NH–CO–O–, –O–CO–NH–, –NH–CO–NH–, wobei endständige –CO-Einheiten des Brückengliedes an den Alkyl- oder Alkenylrest $R_1$ geknüpft sind, und

m und n je 1 oder 2 bedeuten.

5. Verfahren nach Anspruch 4, dadurch gekennzeichnet, dass man als Komponente (A) ein Leimungsmittel der Formel

$$A_1-D_2-(CH_2)_{n-1}-X_2-CO-R_2$$

oder

$$A_1-D_2-X_2-CO-NH-R_3$$

eingesetzt, worin

$D_2$ durch Chlor, Brom, Nitro, Amino oder Hydroxyl substituiertes oder unsubstituiertes Phenylen oder Naphthylen,

$R_2$ Alkyl oder Alkenyl mit 5 bis 21 Kohlenstoffatomen,

$R_3$ Alkyl oder Alkenyl mit 6 bis 22 Kohlenstoffatomen,

$X_2$ –O– oder –NH– und

n 1 oder 2 bedeuten und

$A_1$ die die in Anspruch 4 angegebenen Bedeutungen hat.

6. Verfahren nach einem der Ansprüche 1 bis 5, dadurch gekennzeichnet, dass man als Retentionsmittel (B) ein Polyalkylenimin, Epihalogenhydrin-Addukte von Umsetzungsprodukten aus Polyalkylenpolyaminen und aliphatischen Dicarbonsäuren oder von Umsetzungsprodukten aus Polyalkylenpolyaminen, Dicyandiamid und gegebenenfalls unveresterten oder mit Alkanolen veresterten, organischen Dicarbonsäuren, Umsetzungsprodukte aus Dicyandiamid, Formaldehyd, Ammoniumsalzen starker anorganischer Säuren und Alkylendiaminen oder Polyalkylenpolyaminen, kationisch modifizierte Stärken oder Kohlenhydrate aus Johannisbrot- oder Gurarkernmehl, Copolymerisate auf Basis von Polyamid-Aminen oder Umsetzungsprodukte aus Epihalogenhydrinen und polymerisierten Diallylaminen einsetzt.

7. Wässrige Zusammensetzung zur Durchführung des Verfahrens gemäss Anspruch 2, wobei das Leimungsmittel (A) und das Retentionsmittel (B) in beliebiger Reihenfolge separat zur Faserstoffsuspension gegeben werden, dadurch gekennzeichnet, dass sie das Leimungsmittel (A), mindestens teilweise in Form von Salzen, und gegebenenfalls übliche Zusätze enthält.

8. Wässrige Zusammensetzung zur Durchführung des Verfahrens gemäss Anspruch 2, wobei das Leimungsmittel (A) und das Retentionsmittel (B) gleichzeitig zur Faserstoffsuspension gegeben werden, dadurch gekennzeichnet, das sie (A) 2 bis 40 Gewichtsprozent Leimungsmittel und (B) 0,1 bis 20 Gewichtsprozent Retentionsmittel, bezogen jeweils auf Trockensubstanz von (A) und (B) und auf das Gesamtgewicht der wässrigen Zusammensetzung, und gegebenenfalls übliche Zusätze enthält.

9. Wässrige Leimflotte zur Durchführung des Verfahrens gemäss Anspruch 3, dadurch gekennzeichnet, dass sie (A) 0,2 bis 0,4 Gewichtsprozent Leimungsmittel und (B) 0,01 bis 0,2 Gewichtsprozent Retentionsmittel, bezogen jeweils auf Trockensubstanz von (A) und (B) und auf das Gesamtgewicht der wässrigen Zusammensetzung, und gegebenenfalls übliche Zusätze enthält.

10. Nach dem Verfahren gemäss einem der Ansprüche 1 bis 6 geleimtes Papier oder geleimter Karton.

11. Verwendung der Komponente (A) gemäss einem der Ansprüche 1 bis 5 zum Leimen von Papier oder Karton.

12. Verbindungen der Formel

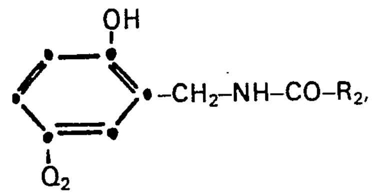
,

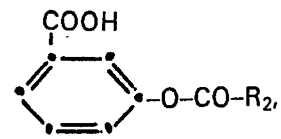
,

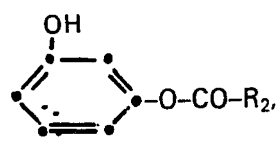
,

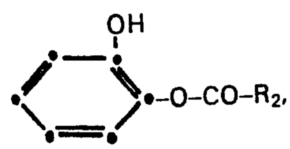
,

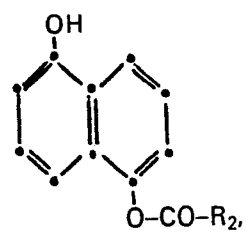
,

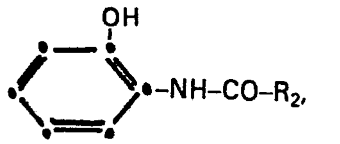

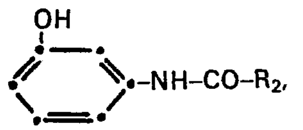

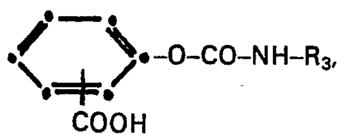

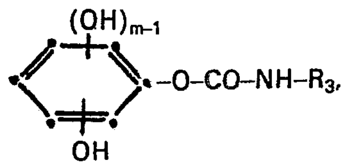

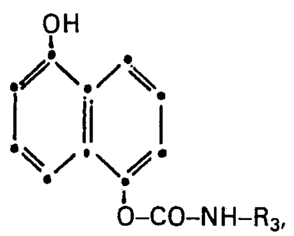

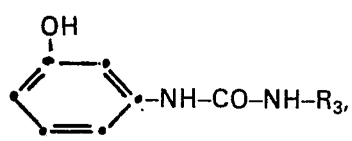

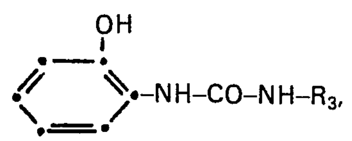

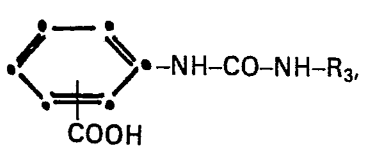

oder

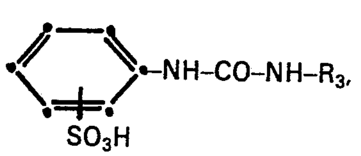

oder deren Salze, worin $Q_2$ Nitro oder Amino, $R_2$ Alkyl oder Alkenyl mit 5 bis 21 Kohlenstoffatomen, $R_3$ Alkyl oder Alkenyl mit 6 bis 22 Kohlenstoffatomen und m 1 oder 2 bedeuten.

13. Verfahren zur Herstellung der Verbindungen gemäss Anspruch 12, dadurch gekennzeichnet, dass man ein methyloliertes Fettsäureamid der Formel

$$R_2\text{–CO–NH–CH}_2\text{OH},$$

worin $R_2$ Alkyl oder Alkenyl mit 5 bis 21 Kohlenstoffatomen bedeutet mit p-Amino- oder p-Nitrophenol, eine Fettsäure der Formel

$$R_2\text{–COOH}$$

oder deren Halogenid, worin $R_2$ Alkyl oder Alkenyl mit 5 bis 21 Kohlenstoffatomen bedeutet, mit 3-Hydroxybenzoesäure, Resorcin, Brenzkatechin, 1,5-Dihydroxynaphthalin, oder mit o- oder m-Aminophenol, oder ein Alkyl- oder Alkenylisocyanat der Formel

$$R_3\text{–N=C=O},$$

worin $R_3$ Alkyl oder Alkenyl mit 6 bis 22 Kohlenstoffatomen bedeutet, mit 2-, 3- oder 4-Hydroxybenzoesäure, Brenzkatechin, Resorcin, Hydrochinon, Phloroglucin, Hydroxyhydrochinon, Pyrogallol, 1,5-Dihydroxynaphthalin oder mit 2- oder 3-Aminophenol, 2-, 3- oder 4-Aminobenzoesäure oder Anilin-2-, -3- oder -4-Sulfonsäure umsetzt.

## Revendications

1. Procédé d'encollage de papier ou de carton, caractérisé en ce qu'on utilise au moins

(A) un agent d'encollage, qui est constitué d'un composé aromatique, lequel présente, sur le noyau aromatique, un substituant hydrophobe unique ayant au moins 5 atomes de carbone, et au moins un groupe anionique, acide ou présent sous forme d'un sel, le substituant hydrophobe étant lié au noyau aromatique par l'intermédiaire d'un chaînon pontant ester, amide, uréthanne ou urée, l'unité CO, dans le cas des chaînons pontants ester et amide, étant liée au radical hydrophobe, et un groupe méthylène étant éventuellement présent entre les chaînons pontants et le noyau aromatique, et

(B) un agent de rétention cationique polymère.

2. Procédé selon la revendication 1, caractérisé en ce que, pour l'encollage de grandes quantités de papier ou de carton, on ajoute les composants (A) et (B), dans un ordre quelconque ou simultanément, aux suspensions aqueuses de fibres, contenant de la cellulose et éventuellement des charges.

3. Procédé selon la revendication 1, caractérisé en ce que, pour l'encollage superficiel du papier, on imprègne le papier avec un bain d'encollage aqueux contenant les composants (A) et (B), et on le sèche.

4. Procédé selon l'une des revendications 1 à 3, caractérisé en ce qu'on utilise comme composant (A) un agent d'encollage de formule

$$\begin{matrix}(A_2)_{m-1} \\ | \\ A_1\text{–}D_1\text{–}(CH_2)_{n-1}\text{–}X_1\text{–}R_1\end{matrix}$$

dans laquelle

$A_1$ et $A_2$, indépendamment l'un de l'autre, sont chacun un groupe carboxyle, hydroxyle ou sulfo,

anionique, se présentant sous forme d'un sel, ou acide,

$D_1$ est un radical phénylène, naphthylène, dihydronaphthylène ou tétrahydronaphtylène éventuellement substitué par un radical halogéno, nitro, amino ou hydroxyle,

$R_1$ est un radical alkyle ou alcényle ayant de 5 à 22 atomes de carbone,

$X_1$ est un chaînon pontant de formule –O–CO–, –NH–CO–, –NH–CO–O–, –O–CO–NH–, –NH–CO–NH–, où les unités CO terminales du chaînon pontant sont liées au radical alkyle ou alcényle $R_1$, et

m et n valent chacun 1 ou 2.

5. Procédé selon la revendication 4, caractérisé en ce qu'on utilise comme composant (A) un agent d'encollage de formule

$$A_1–D_2–(CH_2)_{n-1}–X_2–CO–R_2$$

ou

$$A_1–D_2–X_2–CO–NH–R_3$$

où

$D_2$ est un radical phénylène ou naphtylène éventuellement substitué par un radical chloro, bromo, nitro, amino ou hydroxyle,

$R_2$ est un radical alkyle ou alcényle ayant de 5 à 21 atomes de carbone,

$R_3$ est un radical alkyle ou alcényle ayant de 6 à 22 atomes de carbone,

$X_2$ est –O– ou –NH–, et

n vaut 1 ou 2, et

$A_1$ a les significations données dans la revendication 4.

6. Procédé selon l'une des revendications 1 à 5, caractérisé en ce qu'on utilise comme agent de rétention une polyalkylènimine, des produits obtenus par addition d'épihalogènhydrine à des produits de la réaction de polyalkylène-polyamines et d'acides dicarboxyliques aliphatiques, ou à des produits de la réaction de polyalkylène-polyamines, de dicyanodiamide et éventuellement d'acides dicarboxyliques organiques non-estérifiés ou estérifiés par des alcanols, à des produits de réaction de dicyanodiamide, de formaldéhyde, de sels d'ammonium d'acides inorganiques forts et d'alkylène-diamines ou de polyalkylène-polyamines, des amidons ou hydrates de carbone, à modification cationique, dérivant de la farine de caroube ou de la farine de guar, des copolymères à base de polyamide-amines ou de produits de réaction d'épihalogènhydrines et de diallylamines polymérisées.

7. Composition aqueuse pour la mise en œuvre du procédé selon la revendication 2, dans lequel l'agent d'encollage (A) et l'agent de rétention (B) sont ajoutés séparément, dans un ordre quelconque, à la suspension de matières fibreuses, caractérisée en ce qu'elle contient l'agent d'encollage (A), au moins partiellement, sous forme de sels, et éventuellement des additifs habituels.

8. Composition aqueuse pour la mise en œuvre du procédé selon la revendication 2, dans lequel l'agent d'encollage (A) et l'agent de rétention (B) sont ajoutés simultanément à la suspension de matières fibreuses, caractérisée en ce qu'elle contient

(A) de 2 à 40% en poids de l'agent d'encollage, et

(B) de 0,1 à 20% en poids de l'agent de rétention, dans les deux cas par rapport à la matière sèche de (A) et (B) et par rapport au poids total de la composition aqueuse, et éventuellement des additifs habituels.

9. Bain d'encollage aqueux pour la mise en œuvre du procédé selon la revendication 3, caractérisé en ce qu'il contient

(A) de 0,02 à 0,4% en poids de l'agent d'encollage, et

(B) de 0,01 à 0,2% en poids de l'agent de rétention, dans les deux cas, par rapport à la matière sèche de (A) et (B) et au poids total de la composition aqueuse, et éventuellement des additifs habituels.

10. Papier ou carton encollé par le procédé selon l'une des revendications 1 à 6.

11. Utilisation du composant (A) selon l'une des revendications 1 à 5 pour l'encollage du papier ou du carton.

12. Composés de formule

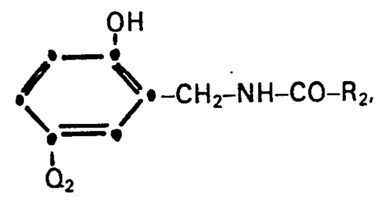

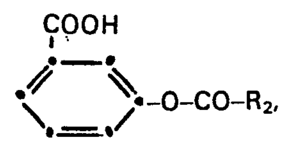

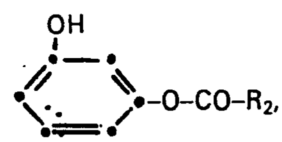

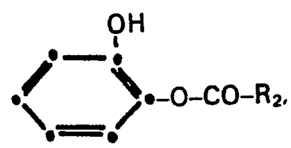

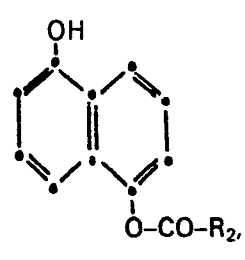

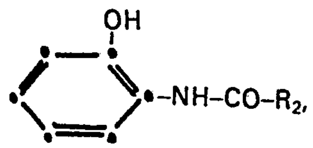

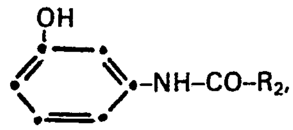

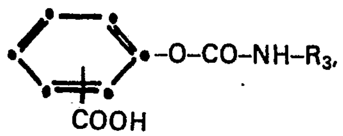

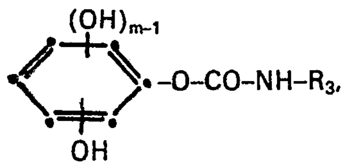

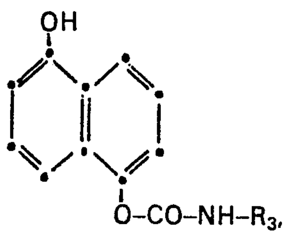

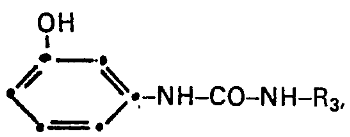

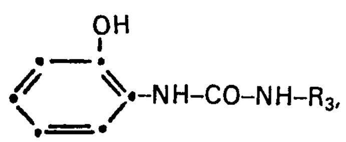

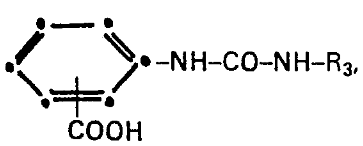

ou

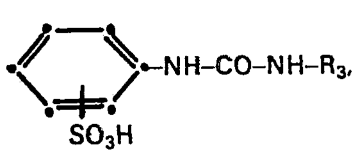

ou leurs sels, où $Q_2$ représente le radical nitro ou amino, $R_2$ est un radical alkyle ou alcényle ayant de 5 à 21 atomes de carbone, $R_3$ est un radical alkyle ou alcényle ayant de 6 à 22 atomes de carbone, et m vaut 1 ou 2.

13. Procédé de préparation des composés selon la revendication 12, caractérisé en ce qu'on fait réagir un amide d'acide gras méthylolé de formule

$$R_2-CO-NH-CH_2OH$$

dans laquelle $R_2$ est un radical alkyle ou alcényle ayant de 5 à 21 atomes de carbone, sur du p-amino- ou du p-nitrophénol; un acide gras de formule

$$R_2-COOH$$

ou l'un de ses halogénures, où $R_2$ est un radical alkyle ou alcényle ayant de 5 à 21 atomes de carbone, sur de l'acide 3-hydroxybenzoïque, du résorcinol, du pyrocatéchol, du 1,5-dihydroxynaphthalène, ou sur de l'o- ou du m-aminophénol; ou un isocyanate d'alkyle ou d'alcényle de formule

$$R_3-N=C=O$$

dans laquelle $R_3$ est un radical alkyle ou alcényle ayant de 6 à 22 atomes de carbone, sur de l'acide 2-, 3- ou 4-hydroxybenzoïque, du pyrocatéchol, du résorcinol, de l'hydroquinone, du phloroglucinol, de l'hydroxyhydroquinone, du pyrogallol, du 1,5-dihydroxynaphtalène, ou sur du 2- où 3-aminophénol, de l'acide 2-, 3- ou 4-aminobenzoïque, ou de l'acide aniline-2-, -3- ou -4-sulfonique.

**Claims**

1. A process for sizing paper or cardboard, which comprises using at least (A) a sizing agent consisting of an aromatic compound which contains in the aromatic nucleus a single hydrophobic substituent having at least 5 carbon atoms and at least one anionic group, acidic or in salt form, the hydrophobic substituent being linked to the aromatic nucleus through an ester, amide, urethane or urea bridge member, in the case of ester and amide bridge members the CO unit being attached to the hydrophobic radical and a methylene group being optionally present between the bridge members and the aromatic nucleus, and (B) a polymeric cationic retention aid.

2. A process according to claim 1, which comprises tub-sizing paper or cardboard by adding components (A) and (B), in any order or simultaneously, to an aqueous cellulose-containing fibre suspension which optionally contains fillers.

3. A process according to claim 1, which comprises surface-sizing paper by impregnating the paper with an aqueous sizing liquor which contains components (A) and (B), followed by drying.

4. A process according to any one of claims 1 to 3, which comprises using, as component (A), a sizing agent of the formula

$$\overset{(A_2)_{m-1}}{\overset{|}{}}$$
$$A_1-D_1-(CH_2)_{n-1}-X_1-R_1$$

in which

$A_1$ and $A_2$ are each independently of one another an anionic carboxyl, hydroxyl or sulfo group which is acidic or in salt form,

$D_1$ is phenylene, naphthylene, dihydronaphthylene or tetrahydronaphthylene, each of which is unsubstituted or substituted by halogen, nitro, amino or hydroxyl,

$R_1$ is alkyl or alkenyl of 5 to 22 carbon atoms,

$X_1$ is a bridge member of the formula $-O-CO-$, $-NH-CO-$, $-NH-CO-O$, $-O-CO-NH-$ or $-NH-CO-NH-$, where terminal $-CO-$ units of the bridge member are attached to the alkyl or alkenyl radical $R_1$, and

m and n are each 1 or 2.

5. A process according to claim 4, which comprises using, as component (A), a sizing agent of the formula

$$A_1-D_2-(CH_2)_{n-1}-X_2-CO-R_2$$

or

$$A_1-D_2-X_2-CO-NH-R_3$$

in which

$D_2$ is phenylene or naphthylene, each unsubstituted or substituted by chlorine, bromine, nitro, amino or hydroxyl,

$R_2$ is alkyl or alkenyl of 5 to 21 carbon atoms,

$R_3$ is alkyl or alkenyl of 6 to 22 carbon atoms,

$X_2$ is $-O-$ or $-NH-$ and

n is 1 or 2 and

$A_1$ is as defined in claim 4.

6. A process according to any one of claims 1 to 5, which comprises using, as retention aid (B), a polyalkylene-imine, epihalohydrin adducts of reaction products of polyalkylenepolyamines and aliphatic dicarboxylic acids or of reaction products of polyalkylenepolyamines, dicyandiamide and unesterified or alkanol-esterified organic dicarboxylic acids, reaction products of dicyandiamide, formaldehyde, ammonium salts of strong inorganic acids and alkylenediamines or polyalkylenepolyamines, cationically modified starches or carbohydrates from carob bean gum or guar gum, copolymers based on polyamide-amines or reaction products of epihalohydrins and polymerised diallyl amines.

7. An aqueous composition for carrying out the process according to claim 2 by adding the sizing agent (A) and the retention aid (B), in any order, separately to the fibre suspension, which composition contains the sizing agent (A), at least partly in salt form, and optionally conventional auxiliaries.

8. An aqueous composition for carrying out the process according to claim 2 by adding the sizing agent (A) and the retention aid (B) simultaneously to the fibre suspension, which composition contains

(A) 2 to 40 percent by weight of sizing agent and

(B) 0.1 to 20 percent by weight of retention aid,

both based on dry substance of (A) and (B) and on the total weight of the aqueous composition, and optionally conventional auxiliaries.

9. An aqueous sizing liquor for carrying out the process according to claim 3, which liquor contains

(A) 0.2 to 0.4 percent by weight of sizing agent, and

(B) 0.01 to 0.2 percent by weight to retention aid,

both based on dry substance of (A) and (B) and on the total weight of the aqueous composition, and optionally conventional auxiliaries.

10. Paper or cardboard sized by a process according to any one of claims 1 to 6.

11. Use of component (A) according to any one of claims 1 to 5 for sizing paper or cardboard.

12. A compound of the formula

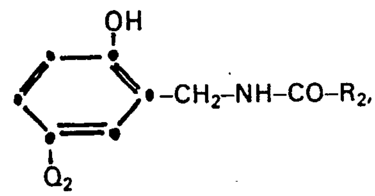

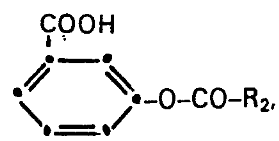

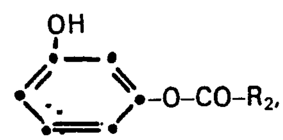

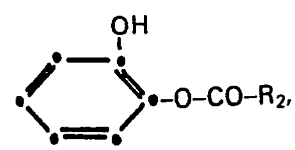

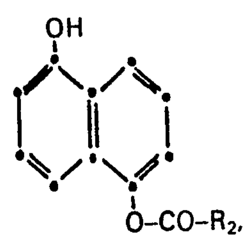

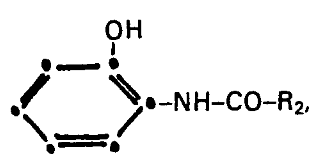

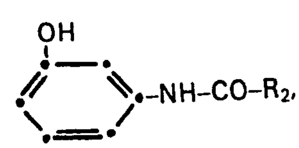

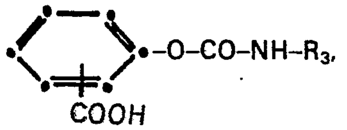

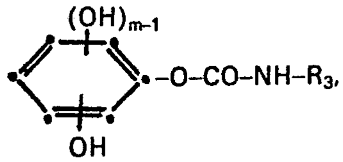

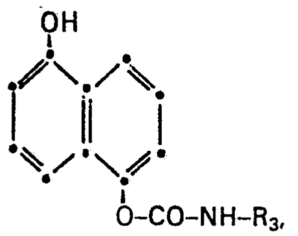

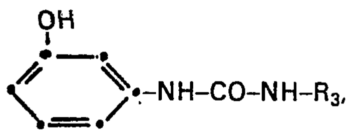

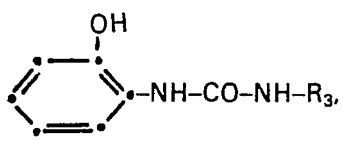

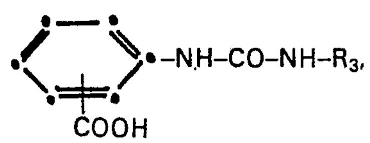

or

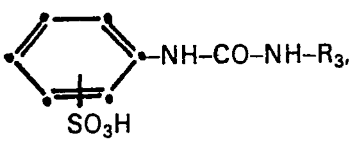

or a salt thereof,
in which
$Q_2$ is nitro or amino,
$R_2$ is alkyl or alkenyl of 5 to 21 carbon atoms,
$R_3$ is alkyl or alkenyl of 6 to 22 carbon atoms and
m is 1 or 2.

13. A process for the preparation of a compound according to claim 12, which comprises reacting a methylolated fatty acid amide of the formula

$$R_2\text{–CO–NH–}CH_2OH$$

in which $R_2$ is alkyl or alkenyl of 5 to 21 carbon atoms, with p-aminophenol or p-nitrophenol, a fatty acid of the formula

$$R_2\text{–COOH}$$

or a halide thereof, in which $R_2$ is alkyl or alkenyl of 5 to 21 carbon atoms, with 3-hydroxybenzoic acid, resorcinol, pyrocatechol, 1,5-dihydroxynaphthalene, or with o-aminophenol or m-aminophenol, or an alkyl or alkenyl isocyanate of the formula

$$R_3\text{–N=C=O}$$

in which $R_3$ is alkyl or alkenyl, of 6 to 22 carbon atoms, with 2-, 3- or 4-hydroxybenzoic acid, pyrocatechol, resorcinol, hydroquinone, phloroglucinol, hydroxyhydroquinone, pyrogallol, 1,5-dihydroxynaphthalene, or with 2- or 3-aminophenol, 2-, 3- or 4-aminobenzoic acid or aniline-2-, -3- or -4-sulfonic acid.